(19) Europäisches Patentamt
European Patent Office
Office européen des brevets

(11) Publication number: 0 643 048 A2

# (12) EUROPEAN PATENT APPLICATION

(21) Application number: 94111965.3

(22) Date of filing: 01.08.94

(51) Int. Cl.6: C07D 239/60, C07D 405/12, C07D 413/12, A01N 43/54, A01N 43/80

(30) Priority: 12.08.93 JP 219290/93

(43) Date of publication of application: 15.03.95 Bulletin 95/11

(84) Designated Contracting States: BE CH DE ES FR GB IT LI NL

(71) Applicant: NIHON BAYER AGROCHEM K.K.
10-8, Takanawa 4-chome
Minato-ku
Tokyo 108 (JP)

(72) Inventor: Goto, Toshio
214-18, Koganei,
Kokubunji-machi
Shimotsuqa-gun,
Tochigi (JP)
Inventor: Kitagawa, Yoshinori
1085, Ara-machi
Moka-shi, Tochigi (JP)
Inventor: Ito, Seishi
1-39-1, Ekihigashidori
Oyama-shi, Tochigi (JP)
Inventor: Shibuya, Katsuhiko
1425-2, Hitotonoya,
Oh-aza
Oyama-shi,
Tochigi (JP)
Inventor: Mineqishi, Natsuko
1-9-31, Wakaqi-cho
Oyama-shi,
Tochigi (JP)
Inventor: Uwaka, Kazuhiro
1-23-13, Ekihigashidori
Oyama-shi,
Tochigi (JP)
Inventor: Yamaoka, Tatsuya
934-7, Hitotonoya,
Oh-aza
Oyama-shi,
Tochigi (JP)
Inventor: Ueno, Chieko
934-7, Hitotonoya,
Oh-aza
Oyama-shi,
Tochigi (JP)
Inventor: Kyo, Yoshiko
934-7, Hitotonoya,
Oh-aza
Oyama-shi,
Tochigi (JP)

(74) Representative: Schumacher, Günter, Dr. et al
Bayer AG
Konzernverwaltung RP
Patentabteilung
D-51368 Leverkusen
Bayerwerk (DE)

## (54) Cyano-containing pyrimidine derivatives and their use as herbicides.

(57) The present invention relates to novel cyano-containing pyrimidine derivatives of the general formula (I)

EP 0 643 048 A2

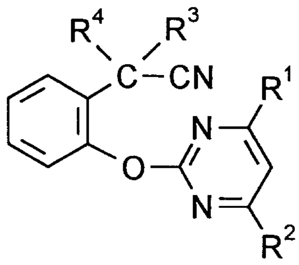

(I)

wherein

- $R^1$ represents halogen, $C_{1-4}$-alkyl, $C_{1-4}$-alkoxy, halogeno-$C_{1-4}$-alkyl, or halogeno-$C_{1-4}$-alkoxy,
- $R^2$ represents halogen, $C_{1-4}$-alkoxy, halogeno-$C_{1-4}$-alkyl, or halogeno-$C_{1-4}$-alkoxy,
- $R^3$ represents halogen, hydroxy, or an oxo group formed together with $R^4$ or
- $R^3$ represents one of the following groups represented by

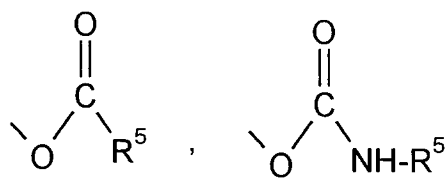

and-O-$SO_2$-$R^6$,

- $R^4$ represents hydrogen or an oxo group formed together with $R^3$,
- $R^5$ represents hydrogen or in each case an optionally substituted or unsubstituted $C_{1-20}$ -alkyl, $C_{2-20}$-alkenyl, $C_{2-20}$-alkynyl, $C_{1-4}$-alkoxycarbonyl, $C_{3-7}$-cycloalkyl, or carboxyl or phenyl,
- $R^5$ represents an optionally substituted or unsubstituted five- or six-membered heterocyclic ring, or an optionally substituted bicyclic group formed by condensation of a five or six-membered heterocyclic ring with phenyl, and
- $R^6$ represents in each case an optionally substituted or unsubstituted $C_{1-20}$-alkyl, $C_{2-20}$-alkenyl, $C_{2-20}$-alkynyl or phenyl;

to processes for their preparation and to their use as herbicides.

The present invention relates to novel cyano-containing pyrimidine derivatives, to processes for their preparation and to their use as herbicides.

It is already been known that a certain kind of pyrimidine derivatives exhibit herbicidal activities (see Japanese Patent Application Disclosure Nos. Sho. 62-174059, Hei.1-115870, Hei.3-52873, Hei.4-221372 and Hei.5-70440).

There have now been found novel cyano-containing pyrimidine derivatives of the general formula (I)

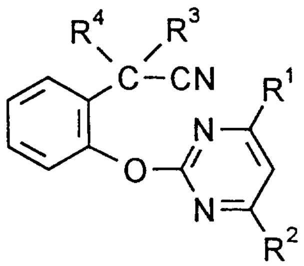

(I)

wherein

$R^1$ represents halogen, $C_{1-4}$-alkyl, $C_{1-4}$-alkoxy, halogeno-$C_{1-4}$-alkyl, or halogeno-$C_{1-4}$-alkoxy,

$R^2$ represents halogen, $C_{1-4}$-alkoxy, halogeno-$C_{1-4}$-alkyl, or halogeno-$C_{1-4}$-alkoxy,

$R^3$ represents halogen, hydroxy, or an oxo group formed together with $R^4$ or

$R^3$ represents one of the following groups represented by

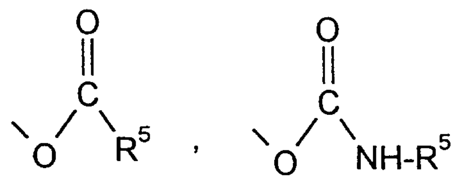

and $-O-SO_2-R^6$,

$R^4$ represents hydrogen or an oxo group formed together with $R^3$,

$R^5$ represents hydrogen or in each case an optionally substituted or unsubstituted $C_{1-20}$-alkyl, $C_{2-20}$-alkenyl, $C_{2-20}$-alkynyl, $C_{1-4}$-alkoxycarbonyl, $C_{3-7}$-cycloalkyl, phenyl, naphthyl or carboxyl or

$R^5$ represents an optionally substituted or unsubstituted five- or six-membered heterocyclic ring, or an optionally substituted bicyclic group formed by condensation of a five or six-membered heterocyclic ring with phenyl, and

$R^6$ represents in each case an optionally substituted or unsubstituted $C_{1-20}$-alkyl, $C_{2-20}$-alkenyl, $C_{2-20}$-alkynyl or phenyl.

Cyano-containing pyrimidine derivatives of the general formula (I) are obtained when

a) in the case where $R^3$ represents a hydroxyl group, and $R^4$ represents a hydrogen atom:

a compound represented by the general formula,

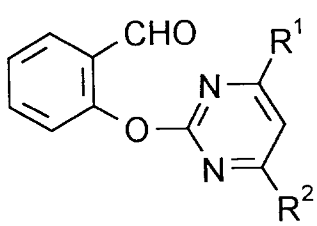

(II)

wherein

$R^1$ and $R^2$ have the same meanings as mentioned above,

is reacted with sodium cyanide or potassium cyanide, if appropriate in the presence of acidic catalysts and if appropriate in the presence of inert solvents,

or

b) in the case where $R^3$ and $R^4$ together form an oxo group: a compound represented by the general formula,

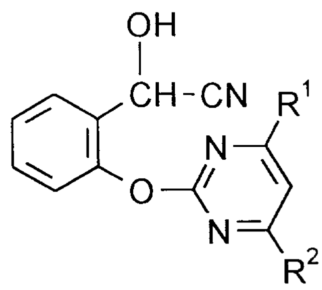

(Ia)

wherein

$R^1$ and $R^2$ have the same meanings as mentioned above,

is oxidized, if appropriate in the presence of inert solvents, or

c) in the case where $R^3$ represents $-O-SO_2-R^6$ and $R^4$ represents a hydrogen atom:

a compound represented by the formula (Ia) is reacted with a compound represented by the general formula

$R^6SO_2R^7$ (III)

wherein $R^6$ has the same meaning as mentioned above and $R^7$ represents an eliminable group such as chloro or bromo, if appropriate in the presence of acid binder and if appropriate in the presence of inert solvents,

or

d) in the case where $R^3$ represents

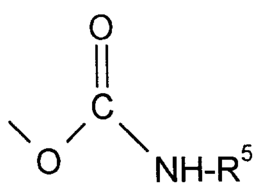

and $R^4$ represents a hydrogen atom:

a compound represented by formula (Ia) is reacted with a compound represented by the general formula

$R^5NCO$ (IV)

wherein $R^5$ has the same meaning as mentioned above,

if appropriate in the presence of acid binder and if appropriate in the presence of inert solvents,

or

e) in the case where $R^3$ represents

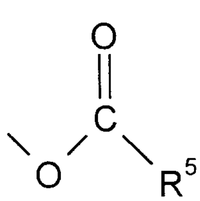

and $R^4$ represents a hydrogen atom:

a compound represented by formula (Ia) is reacted with a compound represented by the general formula

$R^5COR^7$ (V)

wherein $R^5$ and $R^7$ have the same meanings as mentioned above,
if appropriate in the presence of acid binder and if appropriate in the presence of inert solvents,

or

f) in the case where $R^3$ represents

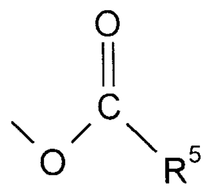

and $R^4$ represents a hydrogen atom:
a compound represented by formula (Ia) is reacted with a compound represented by the general formula

$(R^5CO)_2O$ (VI)

wherein $R^5$ and $R^7$ have the same meanings as mentioned above,
if appropriate in the presence of acid binder and if appropriate in the presence of inert solvents,

or

g) in the case where $R^3$ represents chloro or bromo and $R^4$ represents a hydrogen atom:
a compound represented by formula (Ia) is reacted with thionyl chloride, phosgene, oxal chloride, or a compound represented by the general formula

$R^5SO_2R^8$ (VII)

wherein $R^5$ has the same meaning as mentioned above, and $R^8$ represents chloro or bromo,
if appropriate in the presence of acid binder and if appropriate in the presence of inert solvents.

The compounds represented by the general formula (I) exhibit a strong herbicidal activity.

Unexpectedly and surprisingly there has been found the fact that the pyrimidine derivatives represented by the general formula (I) according to the present invention exhibit substantially far superior herbicidal activities to the compounds that are disclosed by the above-mentioned Japanese Patent Application Disclosures Nos. Sho-62-174059, Sho.63-115870, Hei-3-52873, Hei-4-221372 and Hei-5-70440.

Referring to the compounds represented by the general formula (I) according to the present invention as well as the respective general formula of the intermediate compounds employed in the preparation thereof, the $C_{1-4}$-alkyl group and the alkyl moiety each of the $C_{1-4}$-alkoxy group, halogeno-$C_{1-4}$-alkyl or halogeno-$C_{1-4}$-alkoxy group represent methyl, ethyl, n-propyl, isopropyl, or n-(iso-, sec-, or tert-)butyl, while the halogen atom and the halogen atom of halogeno-$C_{1-4}$-alkyl or halogeno-$C_{1-4}$-alkoxy represents fluoro, chloro, bromo or iodo, preferably fluoro or chloro and, further, the $C_{3-7}$-cycloalkyl represents cyclopropyl, cyclobutyl, cyclopentyl, cyclohexyl and so on, preferably cyclopropyl, cyclopentyl or cyclohexyl.

Referring to the general formula (I) according to the present invention may be mentioned, as the preferred examples, the following compounds or their salts wherein

$R^1$ represents halogen, $C_{1-3}$-alkyl, $C_{1-3}$-alkoxy, halogeno-$C_{1-3}$-alkyl or halogeno-$C_{1-3}$-alkoxy,
$R^2$ represents halogen, $C_{1-3}$-alkoxy, halogeno-$C_{1-3}$-alkyl or halogeno-$C_{1-3}$-alkoxy,
$R^3$ represents chloro, bromo, hydroxy, or an oxo group formed together with $R^4$ or
$R^3$ represents one of the following groups represented by

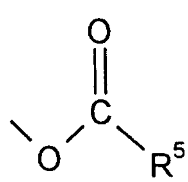

,

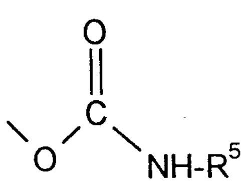

and $-O-SO_2-R^6$,

$R^4$ represents hydrogen, or an oxo group formed together with $R^3$,

$R^5$ represents hydrogen, $C_{1-15}$-alkyl, halogeno-$C_{1-4}$-alkyl, $C_{1-4}$-alkoxy-$C_{1-4}$-alkyl, $C_{3-7}$-cycloalkyl-$C_{1-4}$-alkyl, wherein said cycloalkyl may be substituted by $C_{1-4}$-alkyl or carboxyl, or

$R^5$ further represents cyano-$C_{1-4}$-alkyl, or phenyl-$C_{1-4}$-alkyl wherein said phenyl may be substituted by a halogen or $C_{1-4}$-alkyl,

$R^5$ further represents phenoxy-$C_{1-4}$-alkyl, wherein said phenoxy may be substituted by halogen or a $C_{1-4}$-alkyl,

$R^5$ further represents phenylthio-$C_{1-4}$-alkyl wherein said phenylthio may be substituted by halogen, or $C_{1-4}$-alkyl,

$R^5$ further represents naphthyl-$C_{1-4}$-alkyl, carboxyl-$C_{1-4}$-alkyl, $C_{1-4}$-alkoxy-carbonyl-$C_{1-4}$-alkyl, or $C_{2-6}$-alkenyl wherein said alkenyl may be substituted by halogen or carboxyl,

$R^5$ further represents $C_{2-6}$-alkynyl, $C_{1-4}$-alkoxy-carbonyl wherein said alkoxy may be substituted by halogen, $C_{1-4}$-alkyl or carboxyl,

$R^5$ further represents $C_{3-7}$-cycloalkyl wherein said cycloalkyl may be substituted by halogen, $C_{1-4}$-alkyl or carboxyl,

$R^5$ further represents phenyl or naphthyl wherein said phenyl may be substituted by halogen, $C_{1-4}$-alkyl, halogeno-$C_{1-4}$-alkyl, $C_{1-4}$-alkoxy, halogeno-$C_{1-4}$-alkoxy, cyano, nitro or carboxyl,

$R^5$ further represents a five- or six-membered heterocyclic ring, wherein said hetero atom may be selected from the group consisting of oxygen, nitrogen and sulfur, while said heterocyclic ring may be substituted by halogen or $C_{1-4}$-alkyl,

$R^5$ further represents a group formed by condensation with a five- or six-membered heterocyclic ring with phenyl, wherein the hetero atom of said heterocyclic ring may be selected from the group consisting of oxygen, nitrogen and sulfur and said bicyclic group may be substituted by halogen, $C_{1-4}$-alkyl, $C_{1-4}$-alkylthio or carboxyl, and

$R^6$ represents a $C_{1-15}$-alkyl, halogeno-$C_{1-4}$-alkyl, $C_{1-4}$-alkoxy-$C_{1-4}$-alkyl, $C_{3-7}$-cycloalkyl-$C_{1-4}$-alkyl wherein said cycloalkyl may be substituted by $C_{1-4}$-alkyl or carboxyl,

$R^6$ further represents cyano-$C_{1-4}$-alkyl or phenyl-$C_{1-4}$-alkyl wherein said phenyl may be substituted by halogen or $C_{1-4}$-alkyl,

$R^6$ further represents phenoxy-$C_{1-4}$-alkyl wherein said phenoxy may be substituted by halogen or $C_{1-4}$-alkyl,

$R^6$ further represents phenylthio-$C_{1-4}$-alkyl, wherein said phenylthio may be substituted by halogen or $C_{1-4}$-alkyl,

$R^6$ further represents naphthyl-$C_{1-4}$-alkyl, carboxyl-$C_{1-4}$-alkyl, $C_{1-4}$-alkoxy-carbonyl-$C_{1-4}$-alkyl, $C_{2-6}$-alkenyl, wherein said alkenyl may be substituted by halogen or carboxyl, or

$R^6$ further represents $C_{2-6}$-alkynyl or phenyl.

Referring to the general formula (I) according to the present invention may be mentioned, as particularly preferred examples the following compounds or their salts wherein

$R^1$ represents methoxy,

$R^2$ represents methoxy,

$R^3$ represents chloro, hydroxy, or an oxo group formed together with $R^4$ or

$R^3$ represents one of the following groups represented by

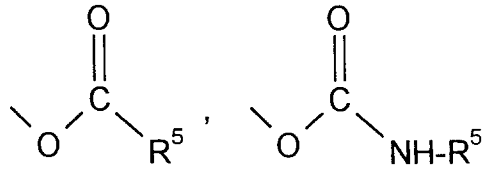

and $-O-SO_2-R^6$,

$R^4$ represents hydrogen or an oxo group formed together with $R^3$,

$R^5$ represents hydrogen, $C_{1-4}$-alkyl, fluoro-$C_{1-4}$-alkyl, chloro-$C_{1-4}$-alkyl, $C_{1-2}$-alkoxy-$C_{1-4}$-alkyl, $C_{3-6}$-cycloalkyl-$C_{1-4}$-alkyl, wherein said cycloalkyl may be substituted by methyl, ethyl, or carboxyl,

$R^5$ further represents cyano-$C_{1-4}$-alkyl, phenyl-$C_{1-4}$-alkyl wherein said phenyl may be substituted by chloro, bromo, or $C_{1-2}$-alkyl,

$R^5$ further represents phenoxy-$C_{1-4}$-alkyl wherein said phenoxy may be substituted by halogen or $C_{1-4}$-alkyl,

$R^5$ further represents phenylthio-$C_{1-4}$-alkyl wherein said phenylthio may be substituted by halogen or $C_{1-4}$-alkyl,

$R^5$ further represents naphthyl-$C_{1-4}$-alkyl, carboxyl-$C_{1-4}$-alkyl, $C_{1-4}$-alkoxy-carbonyl-$C_{1-4}$-alkyl, $C_{2-6}$-alkenyl wherein said alkenyl may be substituted by halogen or carboxyl,

$R^5$ further represents $C_{2-6}$-alkynyl, $C_{1-4}$-alkoxy-carbonyl, wherein said alkoxy may be substituted by halogen, $C_{1-4}$-alkyl or carboxyl,

$R^5$ further represents $C_{3-7}$-cycloalkyl wherein said cycloalkyl may be substituted by halogen, $C_{1-4}$-alkyl or carboxyl,

$R^5$ further represents phenyl or naphthyl wherein said phenyl may be substituted by halogen, $C_{1-4}$-alkyl, halogeno-$C_{1-4}$-alkyl, $C_{1-4}$-alkoxy, halogeno-$C_{1-4}$-alkoxy, cyano, nitro or carboxyl,

$R^5$ further represents a five- or six-membered heterocyclic ring wherein the hetero atom of said heterocyclic ring may be selected from the group consisting of oxygen, nitrogen and sulfur and said heterocyclic ring may be substituted by one of the following substituents: halogen, $C_{1-4}$-alkyl, $C_{1-4}$-alkylthio,

$R^5$ further represents a bicyclic group formed by condensation of a five- or six-membered heterocyclic ring with phenyl, wherein the hetero atom of said bicyclic group may be selected from the group consisting of oxygen, nitrogen and sulfur, and said bicyclic group may be substituted by halogen or $C_{1-4}$-alkyl,

$R^6$ further represents $C_{1-15}$-alkyl, halogeno-$C_{1-4}$-alkyl, $C_{1-4}$-alkoxy-$C_{1-4}$-alkyl, $C_{3-7}$-cycloalkyl-$C_{1-4}$-alkyl wherein said cycloalkyl may be substituted by $C_{1-4}$-alkyl, or carboxyl,

$R^6$ further represents cyano-$C_{1-4}$-alkyl, phenyl-$C_{1-4}$-alkyl, wherein said phenyl may be substituted by halogen or $C_{1-4}$-alkyl,

$R^6$ further represents phenoxy-$C_{1-4}$-alkyl wherein said phenoxy may be substituted by halogen or $C_{1-4}$-alkyl,

$R^6$ further represents phenylthio-$C_{1-4}$-alkyl, wherein said phenylthio may be substituted by halogen or $C_{1-4}$-alkyl,

$R^6$ further represents naphthyl-$C_{1-4}$-alkyl, carboxyl-$C_{1-4}$-alkyl, $C_{1-4}$-alkoxy-carbonyl-$C_{1-4}$-alkyl, or $C_{2-6}$-alkenyl, wherein said alkenyl may be substituted by halogen or carboxyl, or

$R^6$ further represents $C_{2-6}$-alkynyl or phenyl.

The compounds represented by the general formula (I) according to the present invention are shown in the following Table 1:

Table 1

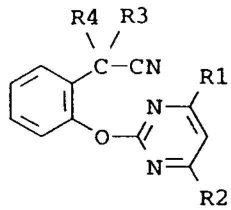

| R1 | R2 | R3 | R4 |
|---|---|---|---|
| $OCH_3$ | $OCH_3$ | OH | H |
| $OCH_3$ | $OCH_3$ | Cl | H |
| $OCH_3$ | $OCH_3$ | O | |
| $OCH_3$ | $OCH_3$ | 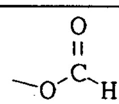 | H |
| $OCH_3$ | $OCH_3$ | 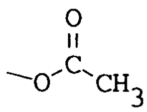 | H |
| $OCH_3$ | $OCH_3$ | 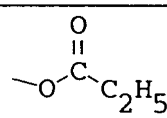 | H |
| $OCH_3$ | $OCH_3$ | 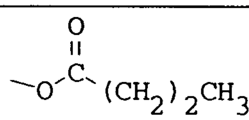 | H |
| $OCH_3$ | $OCH_3$ | 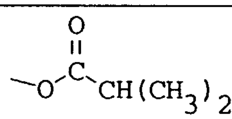 | H |
| $OCH_3$ | $OCH_3$ | 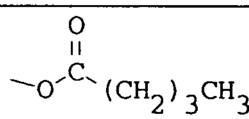 | H |
| $OCH_3$ | $OCH_3$ | 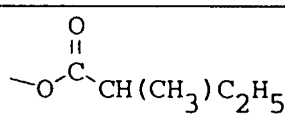 | H |
| $OCH_3$ | $OCH_3$ | 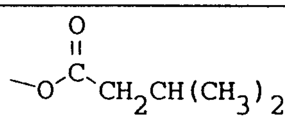 | H |
| $OCH_3$ | $OCH_3$ | 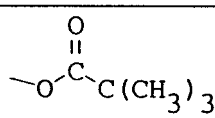 | H |

Table 1 (continued)

| R1 | R2 | R3 | R4 |
|---|---|---|---|
| $OCH_3$ | $OCH_3$ | 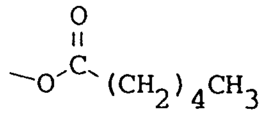 | H |
| $OCH_3$ | $OCH_3$ | 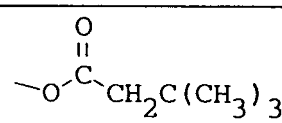 | H |
| $OCH_3$ | $OCH_3$ | 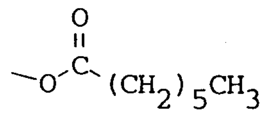 | H |
| $OCH_3$ | $OCH_3$ | 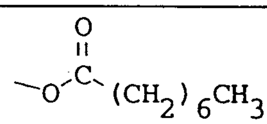 | H |
| $OCH_3$ | $OCH_3$ | 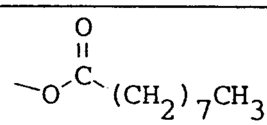 | H |
| $OCH_3$ | $OCH_3$ | 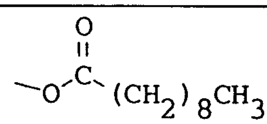 | H |
| $OCH_3$ | $OCH_3$ | 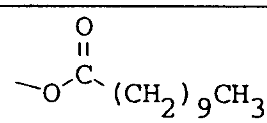 | H |
| $OCH_3$ | $OCH_3$ | 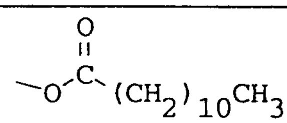 | H |
| $OCH_3$ | $OCH_3$ | 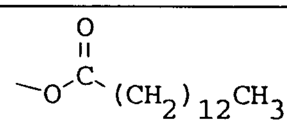 | H |
| $OCH_3$ | $OCH_3$ | 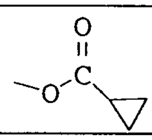 | H |
| $OCH_3$ | $OCH_3$ | 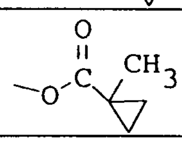 | H |
| $OCH_3$ | $OCH_3$ | 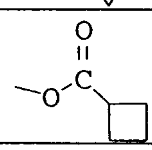 | H |

Table 1 (continued)

| R1 | R2 | R3 | R4 |
|---|---|---|---|
| $OCH_3$ | $OCH_3$ | 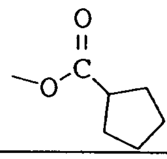 | H |
| $OCH_3$ | $OCH_3$ | 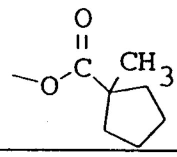 | H |
| $OCH_3$ | $OCH_3$ | 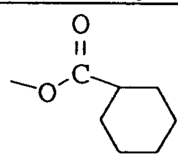 | H |
| $OCH_3$ | $OCH_3$ | 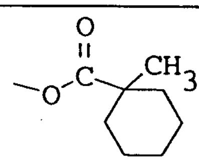 | H |
| $OCH_3$ | $OCH_3$ | 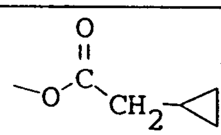 | H |
| $OCH_3$ | $OCH_3$ | 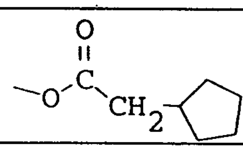 | H |
| $OCH_3$ | $OCH_3$ | 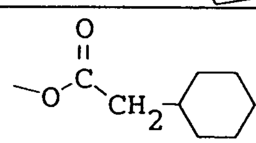 | H |
| $OCH_3$ | $OCH_3$ | 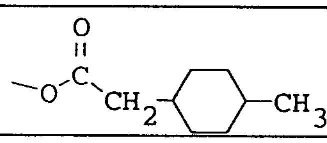 | H |
| $OCH_3$ | $OCH_3$ | 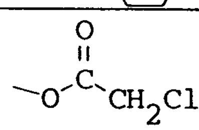 | H |
| $OCH_3$ | $OCH_3$ | 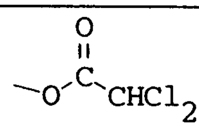 | H |

Table 1 (continued)

| R1 | R2 | R3 | R4 |
|---|---|---|---|
| $OCH_3$ | $OCH_3$ | 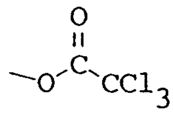 | H |
| $OCH_3$ | $OCH_3$ | 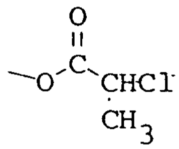 | H |
| $OCH_3$ | $OCH_3$ | 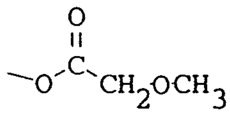 | H |
| $OCH_3$ | $OCH_3$ | 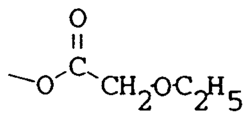 | H |
| $OCH_3$ | $OCH_3$ | 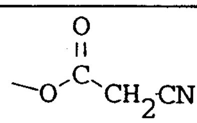 | H |
| $OCH_3$ | $OCH_3$ | 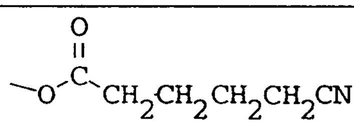 | H |
| $OCH_3$ | $OCH_3$ | 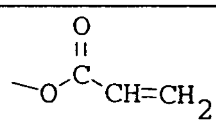 | H |
| $OCH_3$ | $OCH_3$ | 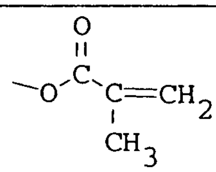 | H |
| $OCH_3$ | $OCH_3$ | 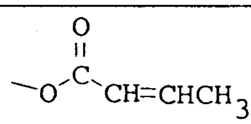 | H |
| $OCH_3$ | $OCH_3$ | 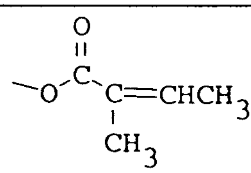 | H |
| $OCH_3$ | $OCH_3$ | 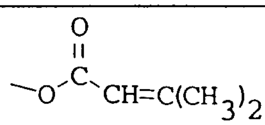 | H |

Table 1 (continued)

| R1 | R2 | R3 | R4 |
| --- | --- | --- | --- |
| $OCH_3$ | $OCH_3$ | 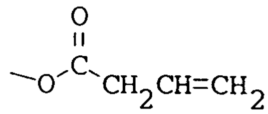 | H |
| $OCH_3$ | $OCH_3$ | 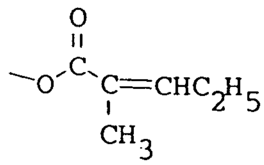 | H |
| $OCH_3$ | $OCH_3$ | 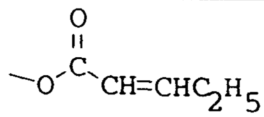 | H |
| $OCH_3$ | $OCH_3$ | 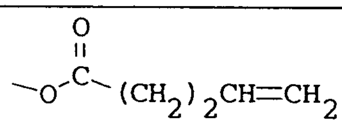 | H |
| $OCH_3$ | $OCH_3$ | 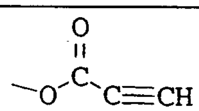 | H |
| $OCH_3$ | $OCH_3$ | 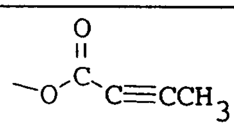 | H |
| $OCH_3$ | $OCH_3$ | 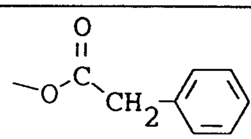 | H |
| $OCH_3$ | $OCH_3$ | 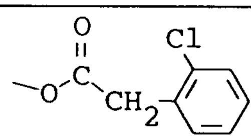 | H |
| $OCH_3$ | $OCH_3$ | 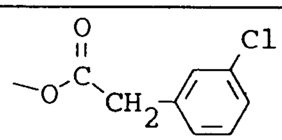 | H |
| $OCH_3$ | $OCH_3$ | 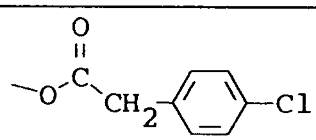 | H |
| $OCH_3$ | $OCH_3$ | 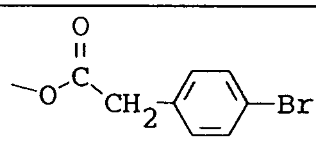 | H |

Table 1 (continued)

| R1 | R2 | R3 | R4 |
|---|---|---|---|
| $OCH_3$ | $OCH_3$ | 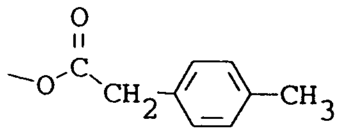 | H |
| $OCH_3$ | $OCH_3$ | 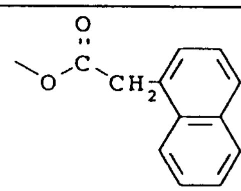 | H |
| $OCH_3$ | $OCH_3$ | 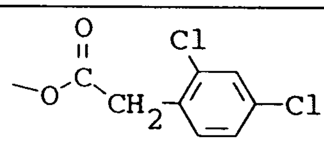 | H |
| $OCH_3$ | $OCH_3$ | 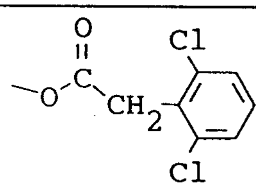 | H |
| $OCH_3$ | $OCH_3$ | 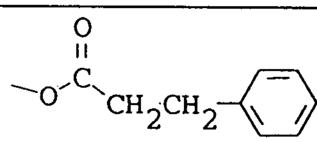 | H |
| $OCH_3$ | $OCH_3$ | 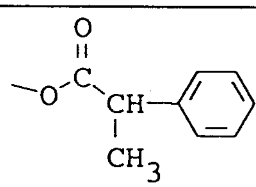 | H |
| $OCH_3$ | $OCH_3$ | 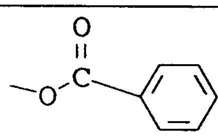 | H |
| $OCH_3$ | $OCH_3$ | 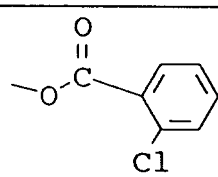 | H |
| $OCH_3$ | $OCH_3$ | 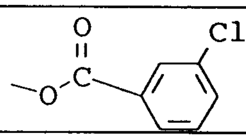 | H |

Table 1 (continued)

| R1 | R2 | R3 | R4 |
|---|---|---|---|
| $OCH_3$ | $OCH_3$ | 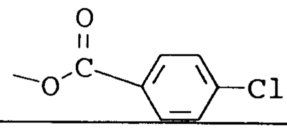 | H |
| $OCH_3$ | $OCH_3$ | 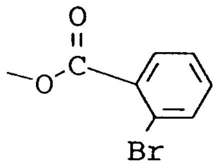 | H |
| $OCH_3$ | $OCH_3$ | 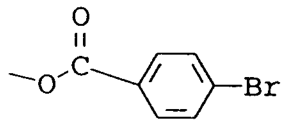 | H |
| $OCH_3$ | $OCH_3$ | 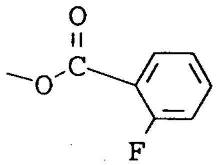 | H |
| $OCH_3$ | $OCH_3$ | 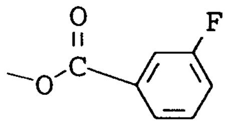 | H |
| $OCH_3$ | $OCH_3$ | 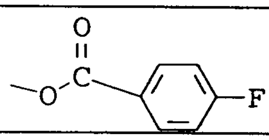 | H |
| $OCH_3$ | $OCH_3$ | 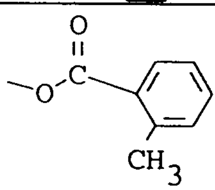 | H |
| $OCH_3$ | $OCH_3$ | 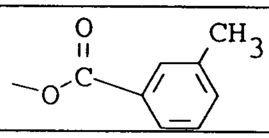 | H |
| $OCH_3$ | $OCH_3$ | 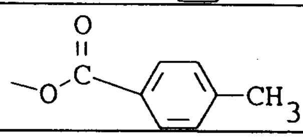 | H |
| $OCH_3$ | $OCH_3$ | 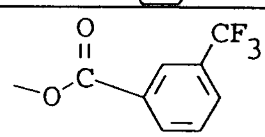 | H |

Table 1 (continued)

| R1 | R2 | R3 | R4 |
|---|---|---|---|
| $OCH_3$ | $OCH_3$ | 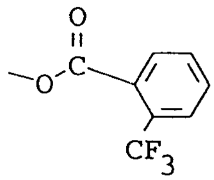 | H |
| $OCH_3$ | $OCH_3$ | 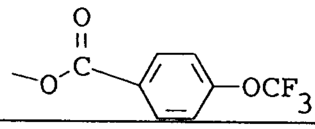 | H |
| $OCH_3$ | $OCH_3$ | 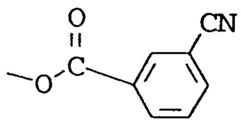 | H |
| $OCH_3$ | $OCH_3$ | 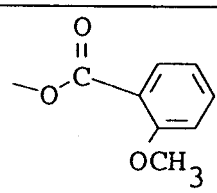 | H |
| $OCH_3$ | $OCH_3$ | 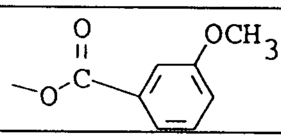 | H |
| $OCH_3$ | $OCH_3$ | 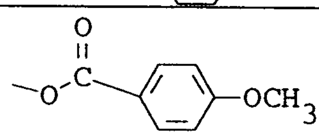 | H |
| $OCH_3$ | $OCH_3$ | 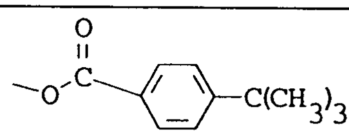 | H |
| $OCH_3$ | $OCH_3$ | 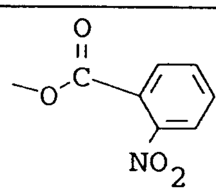 | H |
| $OCH_3$ | $OCH_3$ | 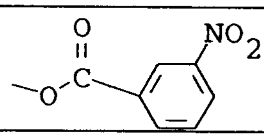 | H |
| $OCH_3$ | $OCH_3$ | 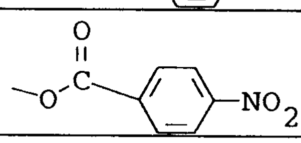 | H |

Table 1 (continued)

| R1 | R2 | R3 | R4 |
|---|---|---|---|
| $OCH_3$ | $OCH_3$ | 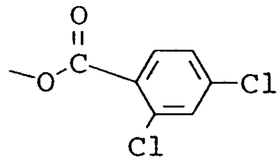 | H |
| $OCH_3$ | $OCH_3$ | 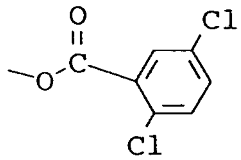 | H |
| $OCH_3$ | $OCH_3$ | 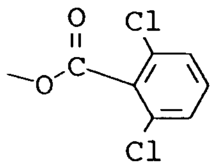 | H |
| $OCH_3$ | $OCH_3$ | 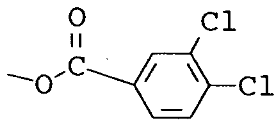 | H |
| $OCH_3$ | $OCH_3$ | 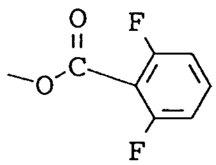 | H |
| $OCH_3$ | $OCH_3$ | 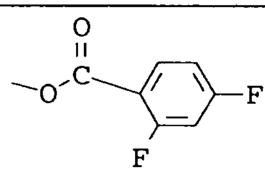 | H |
| $OCH_3$ | $OCH_3$ | 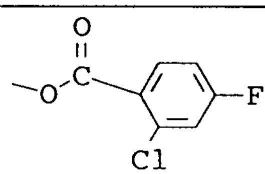 | H |
| $OCH_3$ | $OCH_3$ | 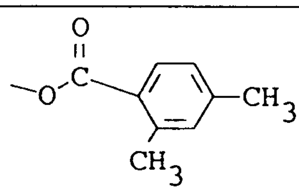 | H |
| $OCH_3$ | $OCH_3$ | 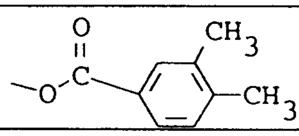 | H |

Table 1 (continued)

| R1 | R2 | R3 | R4 |
|---|---|---|---|
| $OCH_3$ | $OCH_3$ | 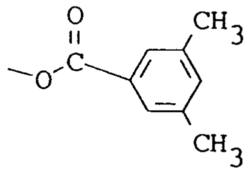 | H |
| $OCH_3$ | $OCH_3$ | 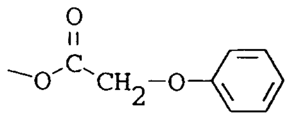 | H |
| $OCH_3$ | $OCH_3$ | 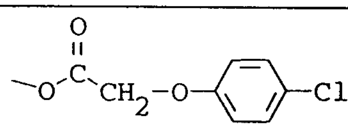 | H |
| $OCH_3$ | $OCH_3$ | 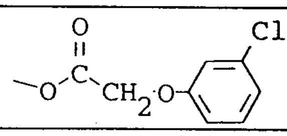 | H |
| $OCH_3$ | $OCH_3$ | 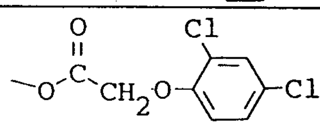 | H |
| $OCH_3$ | $OCH_3$ | 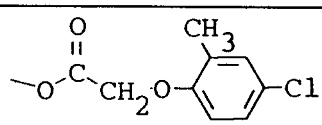 | H |
| $OCH_3$ | $OCH_3$ | 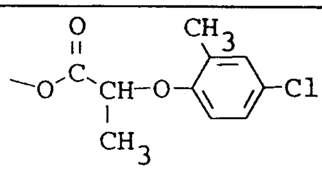 | H |
| $OCH_3$ | $OCH_3$ | 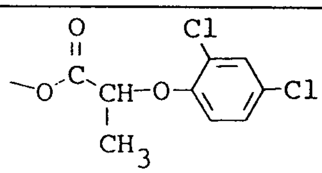 | H |
| $OCH_3$ | $OCH_3$ | 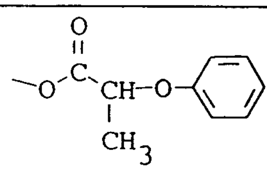 | H |
| $OCH_3$ | $OCH_3$ | 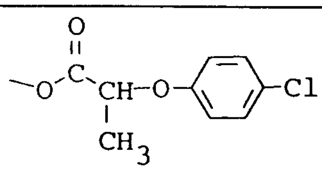 | H |

Table 1 (continued)

| R1 | R2 | R3 | R4 |
|---|---|---|---|
| $OCH_3$ | $OCH_3$ | 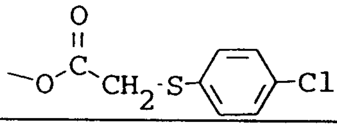 | H |
| $OCH_3$ | $OCH_3$ | 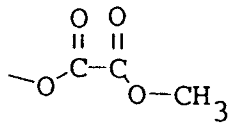 | H |
| $OCH_3$ | $OCH_3$ | 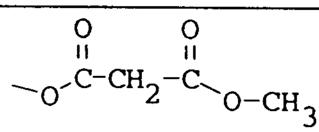 | H |
| $OCH_3$ | $OCH_3$ | 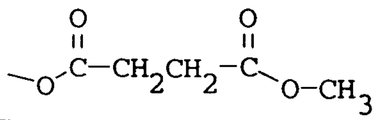 | H |
| $OCH_3$ | $OCH_3$ | 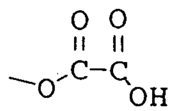 | H |
| $OCH_3$ | $OCH_3$ | 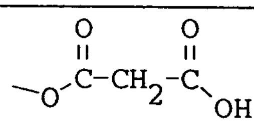 | H |
| $OCH_3$ | $OCH_3$ | 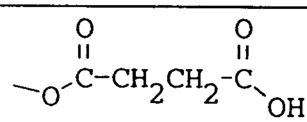 | H |
| $OCH_3$ | $OCH_3$ | 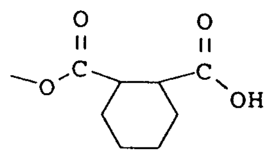 | H |
| $OCH_3$ | $OCH_3$ | 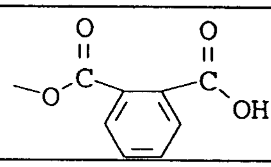 | H |
| $OCH_3$ | $OCH_3$ | 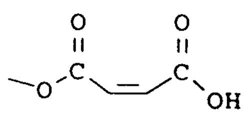 | H |
| $OCH_3$ | $OCH_3$ | 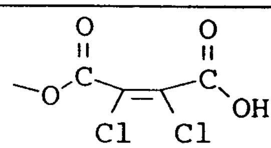 | H |

Table 1 (continued)

| R1 | R2 | R3 | R4 |
|---|---|---|---|
| $OCH_3$ | $OCH_3$ | 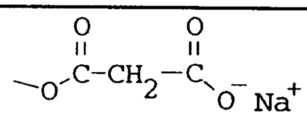 | H |
| $OCH_3$ | $OCH_3$ | 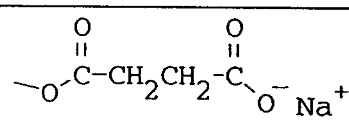 | H |
| $OCH_3$ | $OCH_3$ | 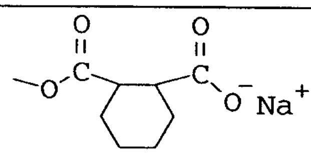 | H |
| $OCH_3$ | $OCH_3$ | 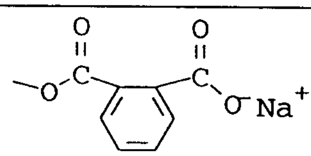 | H |
| $OCH_3$ | $OCH_3$ | 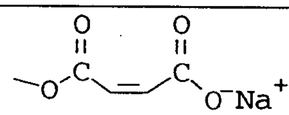 | H |
| $OCH_3$ | $OCH_3$ | 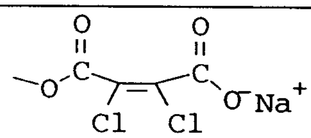 | H |
| $OCH_3$ | $OCH_3$ | 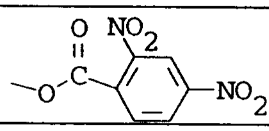 | H |
| $OCH_3$ | $OCH_3$ | 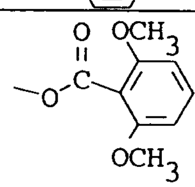 | H |
| $OCH_3$ | $OCH_3$ | 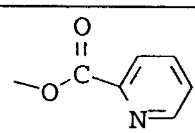 | H |
| $OCH_3$ | $OCH_3$ | 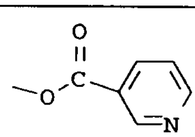 | H |
| $OCH_3$ | $OCH_3$ | 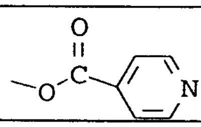 | H |

Table 1 (continued)

| R1 | R2 | R3 | R4 |
|---|---|---|---|
| $OCH_3$ | $OCH_3$ | 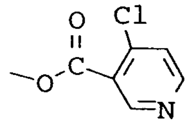 | H |
| $OCH_3$ | $OCH_3$ | 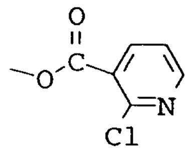 | H |
| $OCH_3$ | $OCH_3$ | 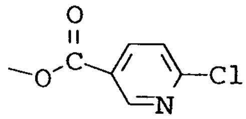 | H |
| $OCH_3$ | $OCH_3$ | 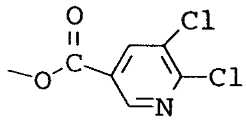 | H |
| $OCH_3$ | $OCH_3$ | 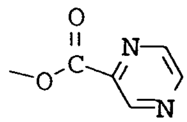 | H |
| $OCH_3$ | $OCH_3$ | 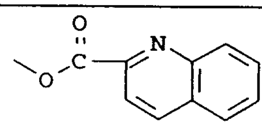 | H |
| $OCH_3$ | $OCH_3$ | 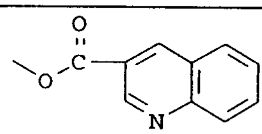 | H |
| $OCH_3$ | $OCH_3$ | 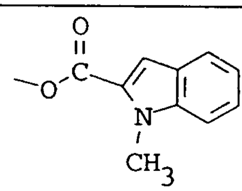 | H |
| $OCH_3$ | $OCH_3$ | 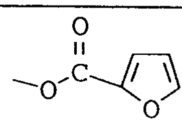 | H |
| $OCH_3$ | $OCH_3$ | 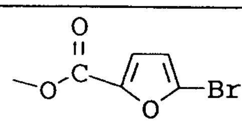 | H |
| $OCH_3$ | $OCH_3$ | 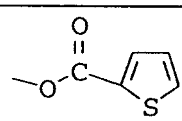 | H |

Table 1 (continued)

| R1 | R2 | R3 | R4 |
|---|---|---|---|
| $OCH_3$ | $OCH_3$ | 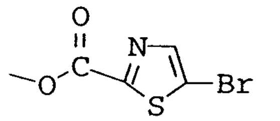 | H |
| $OCH_3$ | $OCH_3$ | 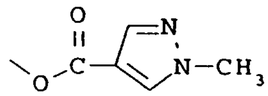 | H |
| $OCH_3$ | $OCH_3$ | 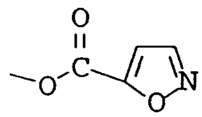 | H |
| $OCH_3$ | $OCH_3$ | 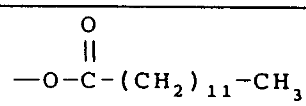 | H |
| $OCH_3$ | $OCH_3$ | 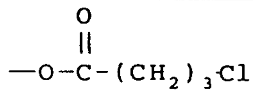 | H |
| $OCH_3$ | $OCH_3$ | 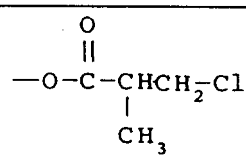 | H |
| $OCH_3$ | $OCH_3$ | 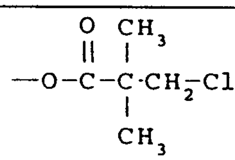 | H |
| $OCH_3$ | $OCH_3$ | 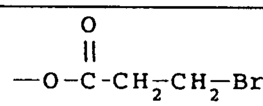 | H |
| $OCH_3$ | $OCH_3$ | 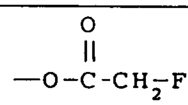 | H |
| $OCH_3$ | $OCH_3$ | 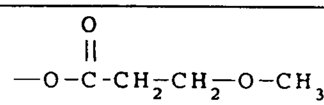 | H |
| $OCH_3$ | $OCH_3$ | 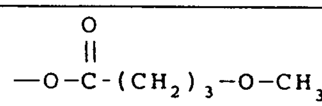 | H |
| $OCH_3$ | $OCH_3$ | 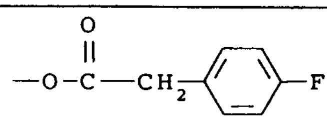 | H |

Table 1 (continued)

| R1 | R2 | R3 | R4 |
|---|---|---|---|
| $OCH_3$ | $OCH_3$ | 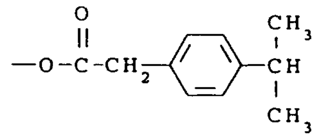 | H |
| $OCH_3$ | $OCH_3$ | 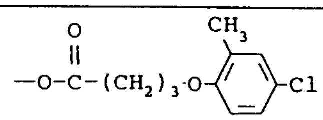 | H |
| $OCH_3$ | $OCH_3$ | 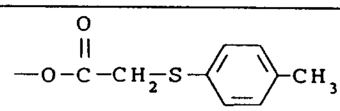 | H |
| $OCH_3$ | $OCH_3$ | 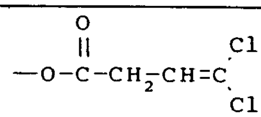 | H |
| $OCH_3$ | $OCH_3$ | 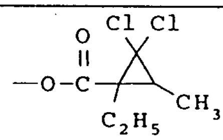 | H |
| $OCH_3$ | $OCH_3$ | 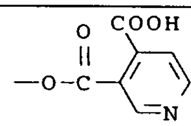 | H |
| $OCH_3$ | $OCH_3$ | 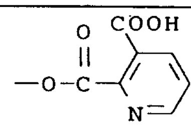 | H |
| $OCH_3$ | $OCH_3$ | 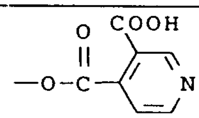 | H |
| $OCH_3$ | $OCH_3$ | 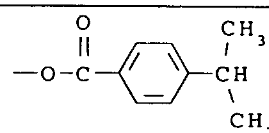 | H |
| $OCH_3$ | $OCH_3$ | 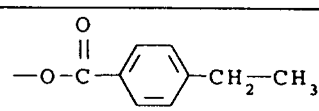 | H |
| $OCH_3$ | $OCH_3$ | 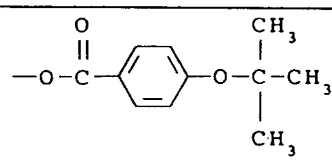 | H |

Table 1 (continued)

| R1 | R2 | R3 | R4 |
|---|---|---|---|
| $OCH_3$ | $OCH_3$ | 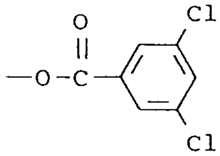 | H |
| $OCH_3$ | $OCH_3$ | 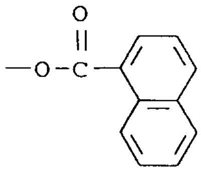 | H |
| $OCH_3$ | $OCH_3$ | 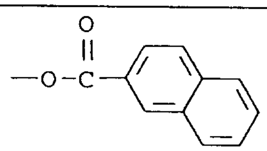 | H |
| $OCH_3$ | $OCH_3$ | 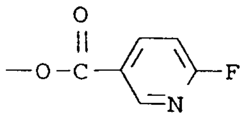 | H |
| $OCH_3$ | $OCH_3$ | 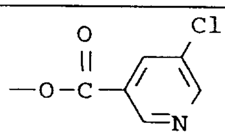 | H |
| $OCH_3$ | $OCH_3$ | 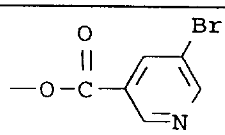 | H |
| $OCH_3$ | $OCH_3$ | 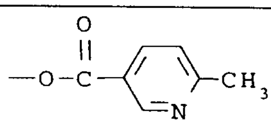 | H |
| $OCH_3$ | $OCH_3$ | 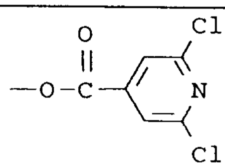 | H |
| $OCH_3$ | $OCH_3$ | 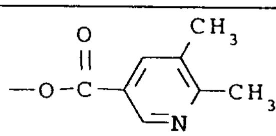 | H |
| $OCH_3$ | $OCH_3$ | 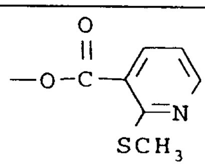 | H |

Table 1 (continued)

| R1 | R2 | R3 | R4 |
|---|---|---|---|
| $OCH_3$ | $OCH_3$ | 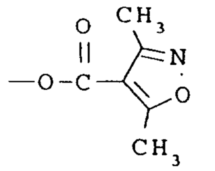 | H |
| $OCH_3$ | $OCH_3$ | 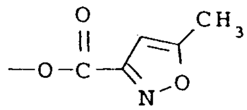 | H |
| $OCH_3$ | $OCH_3$ | 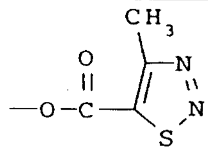 | H |
| $OCH_3$ | $OCH_3$ | 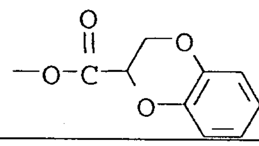 | H |
| | | | |
| $OCH_3$ | $OCH_3$ | 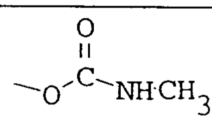 | H |
| $OCH_3$ | $OCH_3$ | 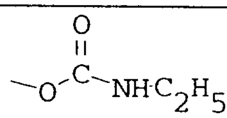 | H |
| $OCH_3$ | $OCH_3$ | 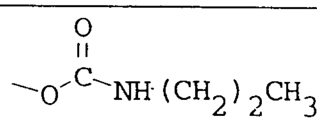 | H |
| $OCH_3$ | $OCH_3$ | 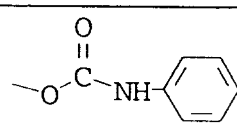 | H |
| $OCH_3$ | $OCH_3$ | 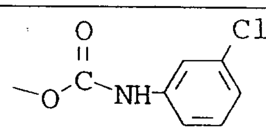 | H |
| $OCH_3$ | $OCH_3$ | 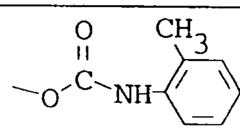 | H |

Table 1 (continued)

| R1 | R2 | R3 | R4 |
|---|---|---|---|
| OCH3 | OCH3 | 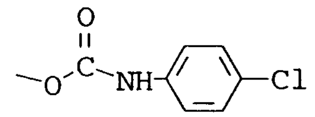 | H |
| OCH3 | OCH3 | 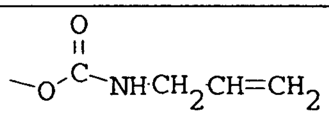 | H |
| OCH3 | OCH3 | $-O-SO_2-CH_3$ | H |
| OCH3 | OCH3 | $-O-SO_2-C_2H_5$ | H |
| OCH3 | OCH3 | $-O-SO_2-(CH_2)_3CH_3$ | H |
| OCH3 | OCH3 | $-O-SO_2-(CH_2)_2CH_3$ | H |
| OCH3 | OCH3 | 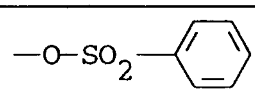 | H |
| OCH3 | OCH3 | 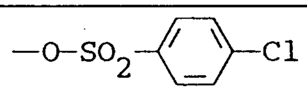 | H |
| OCH3 | OCH3 | 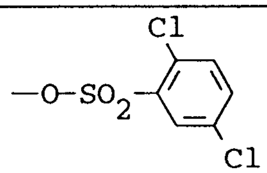 | H |
| OCH3 | OCH3 | 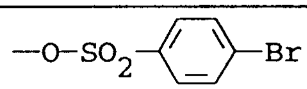 | H |
| OCH3 | OCH3 | 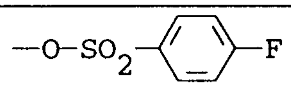 | H |
| OCH3 | OCH3 | $-O-SO_2-CH_2-CH=CH_2$ | H |

In the afore-mentioned process a), if use is made, as starting materials, of 2-(4,6-dimethoxy-2-pyrimidinyloxy)benzaldehyde, potassium cyanide and sodium hydrogen sulfite, for example, the reaction equation can be expressed as follows:

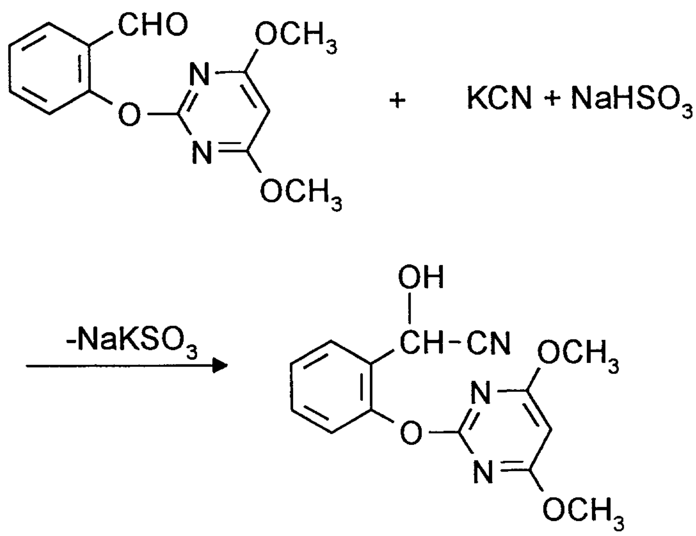


In the afore-mentioned process b), if use is made, as starting materials, of α-hydroxy-2-(4,6-dimethoxy-2-pyrimidinyloxy) phenylacetonitrile and Jones' reagent, for example, the reaction equation can be expressed as follows:

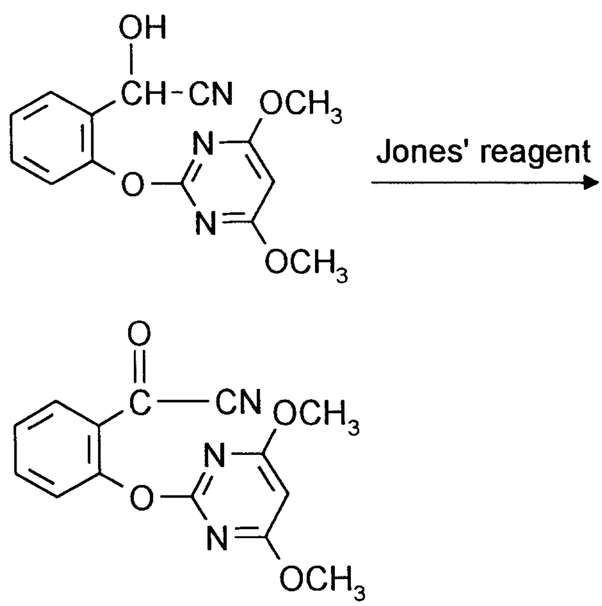


In the afore-mentioned process c), if use is made, as starting materials, of α-hydroxy-2-(4,6-dimethoxy-2-pyrimidinyloxy)phenylacetonitrile and methanesulfonyl chloride, for example, the reaction equation can be expressed as follows:

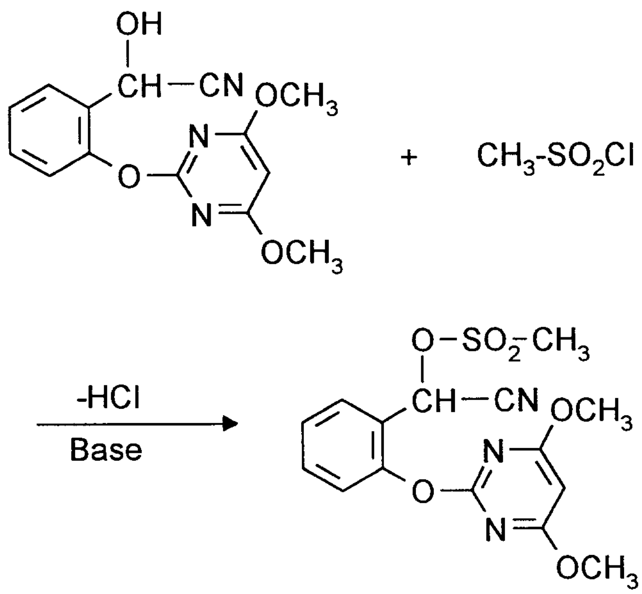


In the afore-mentioned process d), if use is made, as starting materials, of α-hydroxy-2-(4,6-dimethoxy-2-pyrimidinyloxy) phenylacetonitrile and ethylisocyanate, for example, the reaction equation can be expressed as follows:

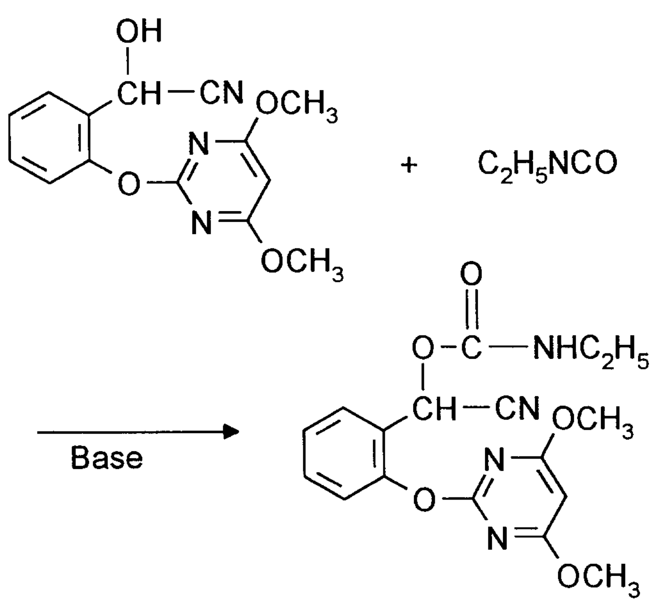


In the afore-mentioned process e), if use is made, as starting materials, of α-hydroxy-2-(4,6-dimethoxy-2-pyrimidinyloxy) phenylacetonitrile and acetylchloride, for example, the reaction equation can be expressed as follows:

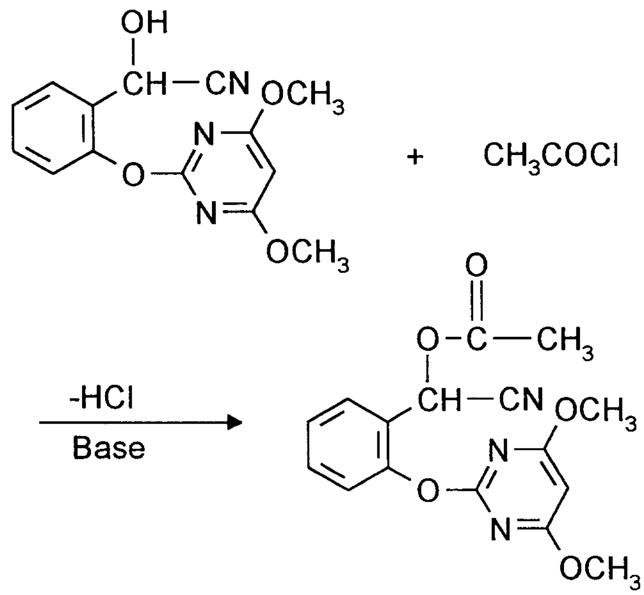


In the afore-mentioned process f), if use is made, as starting materials, of α-hydroxy-2-(4,6-dimethoxy-2-pyrimdinyloxy) phenylacetonitrile and acetic anhydride, for example, the reaction equation can be expressed as follows:

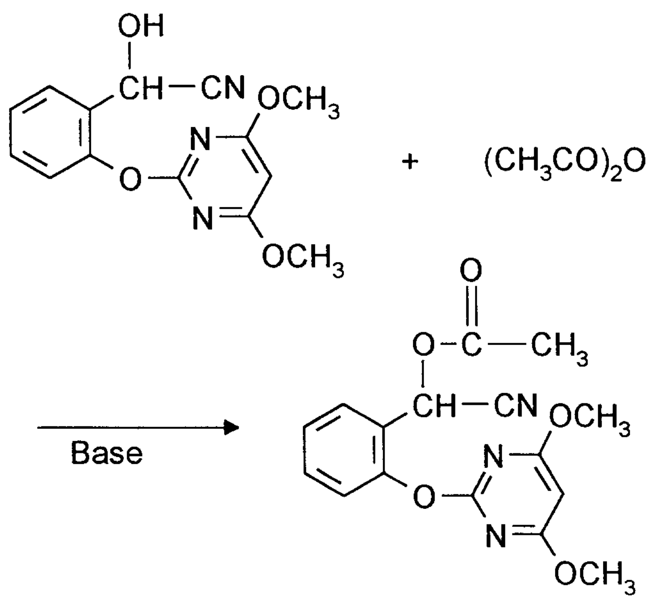


In the afore-mentioned process g), if use is made, as starting materials, of α-hydroxy-2-(4,6-dimethoxy-2-pyrimidinyloxy) phenylacetonitrile and methanesulfonyl chloride in the presence of pyridine, for example, the reaction equation can be expressed as follows:

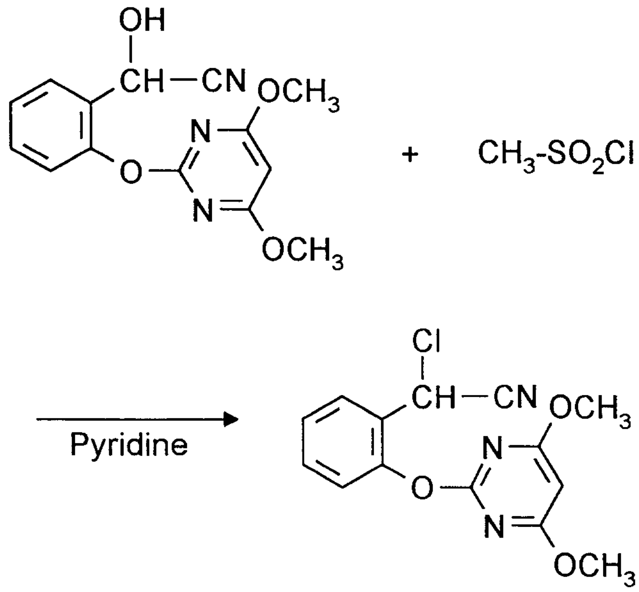


In the afore-mentioned process a), the material compounds represented by the general formula (II) are those having the substituents defined before under $R^1$ and $R^2$, preferably those having the preferred substituents defined before under $R^1$ and $R^2$. The compounds represented by the general formula (II) are known as disclosed in Japanese Patent Application Disclosure No. Sho-62-174059. As the examples of the compounds represented by the general formula (II) may be mentioned the following compounds:
2-(4,6-dimethoxy-2-pyrimidinyloxy) benzaldehyde,
2-(4,6-di(trifluoromethoxy)-2-pyrimidinyloxy) benzaldehyde,
2-(4-dichloromethyl-6-methoxy-2-pyrimidinyloxy) benzaldehyde,
2-(4-chloro-6-methoxy-2-pyrimidinyloxy) benzaldehyde, and
2-(4-difluoromethoxy-6-methoxy-2-pyrimdinyloxy) benzaldehyde.

Other than the above-mentioned process a), there are known a number of synthesizing processes of cyanohydrins such as those disclosed in an article entitled "Organic Compounds, Synthesis and Reaction", Shin Jikken Kagaku Koza No. 14, compiled by Nihon Kagaku Kai, published by Maruzen Co., Ltd.

In the afore-mentioned processes b) to g), the material compounds represented by the general formula (Ia) are those having the substituents defined before under $R^1$ and $R^2$, preferably those having the preferred substituents defined before under $R^1$ and $R^2$. The compounds represented by the general formula (Ia) are compounds which can be prepared according to the afore-mentioned process b) according to the present invention.

As the example of the compounds represented by the general formula (Ia) there may be mentioned the following compound:

α-hydroxy-2-(4,6-dimethoxy-2-pyrimidinyloxy) phenylacetonitrile.

The oxidizing agents employed in the above-mentioned process b) are those which are generally used in organic chemical field as summarized in a publication entitled "Shin Kagaku Jikken Koza 15, Sanka to Kangen" (Lecture of New Chemical Experiment No. 15, Oxidation and Reduction) [I-2], compiled by Nihon Kagaku Kai, published by Maruzen Publishing Co., Ltd.

As the concrete examples of such oxidizing agents may be mentioned:

Jones' reagent,

pyridinium chlorochromate, and

pyridinium dichromate.

In the afore-mentioned process c), the material compounds represented by the general formula (III) are those having the substituents defined before under $R^6$ and $R^7$, preferably those having the preferred substituents defined before under $R^6$ and $R^7$, and particularly preferred the chlorine atom.

The compounds represented by the general formula (III) are well known and generally used in organic chemical field, and, as the examples, can be designated as follows:
methanesulfonylchloride or bromide,
ethanesulfonylchloride or bromide,
benzenesulfonylchloride or bromide, and

p-chlorobenzenesulfonylchloride or bromide.

In the afore-mentioned process d), the material compounds represented by the general formula (IV) are those having the substituents defined before under $R^5$, preferably those having the preferred substituents defined before under $R^5$. The compounds represented by the general formula (IV) are those which are well known and generally used in organic chemical field. As the examples of the compounds represented by the general formula (IV) may be mentioned the following compounds:
ethylisocyanate,
methylisocyanate,
phenylisocyanate, and
p-chlorophenylisocyanate.

In the afore-mentioned process e), the material compounds represented by the general formula (V) are those having the substituents defined before under $R^5$ and $R^7$, preferably those having the preferred substituents defined before under $R^5$ and $R^7$, and particularly preferred the chlorine atom

The compounds represented by the general formula (V) are well known and generally employed in organic chemical field and as the examples of the compounds represented by the general formula (V) may be designated as follows:
benzoyl chloride or bromide,
cinnamyl chloride or bromide,
propionyl chloride or bromide,
phenoxyacetyl chloride or bromide,
nicotinoyl chloride or bromide,
p-chlorobenzoyl chloride or bromide, and
chloroacetyl chloride or bromide.

In the afore-mentioned process f), the compounds represented by the general formula (VI) are those having the substituents defined before under $R^5$ and $R^7$, preferably those having the preferred substituents defined before under $R^5$ and $R^7$, and particularly preferred the chlorine atom.

The compounds represented by the general formula (VI) are well known and generally employed in organic chemical field and as the examples of the compounds of such compounds may be designated as follows:
acetic anhydride,
propionic anhydride, and
formic acid anhydride.

In the afore-mentioned process g), the material compounds represented by the general formula (VII) are those having the substituents defined before under $R^5$ and $R^8$, preferably those having the preferred substituents defined before under $R^5$ and $R^8$, and particularly preferred the chlorine atom.

The compounds represented by the general formula (VII) are well known and generally employed in organic chemical field. As the examples of the compounds represented by the general formula (VII) may be mentioned the following compounds:
methanesulfonyl chloride,
ethanesulfonyl chloride, and
benzenesulfonyl chloride.

In carrying out the process a) mentioned above, use may be made, as suitable diluent, of any inert organic solvent.

Examples of such diluents are water, aliphaltic, cycloaliphatic and aromatic, optionally chlorinated, hydrocarbons such as pentane, hexane, cyclohexane, petroleum ether, ligroin, benzene, toluene, xylene, dichloromethane, chloroform, carbon tetrachloride, 1,2-dichloroethane, ethylenechloride, chlorobenzene, dichlorobenzene, and the like; ethers such as ethyl ether, methyl ethyl ether, isopropyl ether, butyl ether, dioxane, dimethoxyethane (DME), tetrahydrofuran (THF), diethyleneglycol dimethylether (DGM) and the like; nitriles such as acetonitrile, propionitrile, acrylonitrile and the like; alcohols such as methanol, ethanol, isopropanol, butanol, ethyleneglycol and the like; esters such as, for example, ethyl acetate, amylacetate, and the like; acid amides such as dimethyl formamide (DMF), dimethyl acetamide (DMA), N-methylpyrrolidone, 1,3-dimethyl-2-imidazolidinone, hexamethylphosphoric triamide (HMPA) and the like; and sulfones and sulfoxides such as dimethyl sulfoxide (DMSO), sulfolane and the like.

The above-mentioned process a) is carried out preferably in the presence of acidic catalysts and as the acidic catalysts may be mentioned organic amine hydrochloride, sodium hydrogen sulfite, acetic acid and hydrochloric acid.

In the above-mentioned process a), the reaction temperature can be varied within a substantially wide range. In general, the reaction is carried out at a temperature of from about -20 °C to about 80 °C,

preferably from about 0°C to about 35°C.

Further, the reaction is carried out under normal pressure, although it is also possible to employ a higher or reduced pressure.

When the above-mentioned process a) according to the present invention is carried out, use is made, for example, of about 1 to 3 mols amount of sodium cyanide in a diluent such as ether-water per 1 mol of a compound represented by the general formula (II) in the presence of ammonium chloride to obtain the aimed compound.

In carrying out the process b) mentioned above, use may be made, as suitable diluent, of any inert organic solvent.

Examples of such diluents are water, aliphatic, cycloaliphatic and aromatic, optionally chlorinated, hydrocarbons such as pentane, hexane, cyclohexane, petroleum ether, ligroin, benzene, toluene, xylene, dichloromethane, chloroform, carbon tetrachloride, 1,2-dichloroethane, ethylenechloride, chlorobenzene, dichlorobenzene, and the like; ethers such as ethyl ether, methyl ethyl ether, isopropyl ether, butyl ether, dioxane, dimethoxyethane (DME), tetrahydrofuran (THF), diethyleneglycol dimethylether (DGM) and the like; ketones such as acetone, methylethyl ketone (MEK), methyl isopropyl ketone, methyl isobutyl ketone (MIBK), and the like; nitriles such as acetonitrile, propionitrile, acrylonitrile and the like; esters such as, for example, ethyl acetate, amylacetate and the like; acid amides such as dimethyl formamide (DMF), dimethyl acetamide (DMA), N-methyl-pyrrolidone, 1,3-dimethyl-2-imidazolidinone, hexamethylphosphoric triamide (HMPA) and the like; and sulfones and sulfoxides such as dimethyl sulfoxide (DMSO), sulfolane and the like.

In the above-mentioned process b), the reaction temperature can be varied within a substantially wide range. In general, the reaction is carried out at a temperature of from about -10°C to about 50°C, preferably from about 0°C to about 35°C.

Further, the reaction is carried out under normal pressure, although it is also possible to employ a higher or reduced pressure.

When the above-mentioned process b) according to the present invention is carried out, use is made, for example, of about 1 to 3 mols amount of an oxidizing agent such as Jones reagent, for example, in a diluent such as acetone, for example, per 1 mol of a compound represented by the general formula (Ia) in the reaction to obtain the aimed compound.

In carrying out the process c) mentioned above, use may be made, as suitable diluent, of any inert ogranic solvent.

Examples of such diluents are aliphatic, cycloaliphatic and aromatic, optionally chlorinated, hydrocarbons such as pentane, hexane, cyclohexane, petroleum ether, ligroin, benzene, toluene, xylene, dichloromethane, chloroform, carbon tetrachloride, 1,2-dichloroethane, ethylene chloride, chlorobenzene, dichlorobenzene and the like; ethers such as diethyl ether, methyl ethyl ether, di-isopropyl ether, dibutyl ether, dioxane, dimethoxyethane (DME), tetrahydrofuran (THF), diethyleneglycol dimethylether (DGM) and the like; ketones such as acetone, methylethyl ketone (MEK), methyl-isopropyl ketone, methyl isobutyl ketone (MIBK) and the like; nitriles such as acetonitrile, propionitrile, acrylonitrile, and the like; esters such as, for example, ethyl acetate, amyl acetate and the like; acid amides such as dimethyl formamide (DMF), dimethyl acetamide (DMA), N-methylpyrrolidone, 1,3-dimethyl-2-imidazolidinone, hexamethylphosphoric triamide (HMPA) and the like; sulfones and sulfoxides such as dimethyl sulfoxide (DMSO), sulfolane and the like, and bases such a pyridine for example.

The above-mentioned process c) is carried out in the presence of an acid binder and as acid binder may be mentioned inorganic bases such as, for example, hydroxide, carbonate, and bicarbonate of alkalimetals such as, for example, sodium hydrogen carbonate, potassium hydrogen carbonate, sodium carbonate, potassium carbonate, lithium hydroxide, sodium hydroxide, potassium hydroxide, and calcium hydroxide. As inorganic alkalimetal amides may be mentioned, for example, lithium amide, sodium amide, and potassium amide, for example. As organic bases may be mentioned alcolates, tertiary amines, dialkylaminoanilines and pyridines such as, for example, triethylamine, 1,1,4,4-tetramethyl-ethylenediamine (TMEDA), N,N-dimethylaniline, N,N-diethylaniline, pyridine, 4-dimethylamino pyrimidine (DMAP), 1,4-diazabicyclo[2,2,2]octane (DABCO), and 1,8-diazabicyclo[5,4,0]-undec-7-ene (DBU).

Further may be mentioned organic lithium compounds such as, for example, methyl lithium, n-butyl lithium, sec-butyl lithium, tert-butyl lithium, phenyl lithium, dimethylcopper lithium, lithium diisopropylamide, lithium cyclohexylisopropylamide, lithium dicyclohexylamide, n-butyl lithium, DABCO, n-butyl lithium, DBU, and n-butyl lithium, TMEDA, etc.

In the above-mentioned process c), the reaction temperature can be varied within a substantially wide range. In general, the reaction is carried out at a temperature of from about -30°C to about 100°C, preferably from about 0°C to about 35°C.

Further, the reaction is carried out under normal pressure, although it is also possible to employ a higher or reduced pressure.

When the above-mentioned process c) according to the present invention is carried out, use is made, for example, of about 1 to 2 mols amount of a compound selected from the compound (III), in a diluent such as THF per 1 mol of a compound represented by the general formula (Ia) in the presence of triethyl amine to obtain the aimed compound.

In carrying out the process d) mentioned above, use may be made, as suitable diluent, of any inert organic solvent.

Examples of such diluents are aliphatic, cycloaliphatic and aromatic, optionally chlorinated, hydrocarbons such as pentane, hexane, cyclohexane, petroleum ether, ligroin, benzene, toluene, xylene, dichloromethane, chloroform, carbon tetrachloride, 1 ,2-dichloroethane, ethylene chloride, chlorobenzene, dichlorobenzene and the like; ethers such as ethyl ether, methyl ethyl ether, isopropyl ether, butyl ether, dioxane, dimethoxyethane (DME), tetrahydrofuran (THF), diethyleneglycol dimethylether (DGM) and the like; ketones such as acetone, methylethyl ketone (MEK), methyl-isopropyl ketone, methyl-isobutyl ketone (MIBK) and the like; nitriles such as acetonitrile, propionitrile, acrylonitrile, and the like; esters such as, for example, ethyl acetate, amyl acetate and the like; acid amides such as dimethyl formamide (DMF), dimethyl acetamide (DMA), N-methylpyrrolidone, 1,3-dimethyl-2-imidazolidinone, hexamethylphosphoric triamide (HMPA) and the like; sulfones and sulfoxides such as dimethyl sulfoxide (DMSO), sulfolane and the like, and bases such as pyridine for example.

The above-mentioned process d) is carried out in the presence of an acid binder and as the acid binder may be mentioned inorganic bases such as, for example, hydroxide, carbonate, and bicarbonate of alkalimetals such as, for example, sodium hydrogen carbonate, potassium hydrogen carbonate, sodium carbonate, potassium carbonate, lithium hydroxide, sodium hydroxide, potassium hydroxide, and calcium hydroxide. As inorganic alkalimetal amides may be mentioned, for example, lithium amide, sodium amide, and potassium amide, for example. As organic bases may be mentioned tertiary amines, dialkylaminoanilines and pyridines such as, for example, triethylamine, 1,1,4,4-tetramethylethylenediamine (TMEDA), N,N-dimethylaniline, N,N-diethylaniline, pyridine, 4-dimethylamino pyrimidine (DMAP), 1,4-diazabicyclo[2,2,2]-octane (DABCO), and 1,8-diazabicyclo[5,4,0]undec-7-ene (DBU).

Further may be mentioned organic lithium compounds such as, for example, methyl lithium, n-butyl lithium, sec-butyl lithium, tert-butyl lithium, phenyl lithium, dimethylcopper lithium, lithium diisopropylamide, lithium cyclohexylisopropylamide, lithium dicyclohexylamide, n-butyl lithium, DABCO, n-butyl lithium, DBU, and n-butyl lithium, TMEDA, etc.

In the above-mentioned process d), the reaction temperature can be varied within a substantially wide range. In general, the reaction is carried out at a temperature of from about -30 °C to about 150 °C, preferably from about 0 °C to about 50 °C.

Further, the reaction is carried out under normal pressure, although it is also possible to employ a higher or reduced pressure.

When the above-mentioned process d) according to the present invention is carried out, use is made, for example, of about 1 to 2 mols amount of a compound represented by the general formula (IV), in a diluent such as tetrahydrofuran per 1 mol of a compound represented by the general formula (Ia) in the presence of an acid binder such as triethylamine, for example, to obtain the aimed compound.

In carrying out the process e) mentioned above, use may be made, as suitable diluent, of any inert organic solvent.

Examples of such diluents are aliphatic, cycloaliphatic and aromatic, optionally chlorinated, hydrocarbons such as pentane, hexane, cyclohexane, petroleum ether, ligroin, benzene, toluene, xylene, dichloromethane, chloroform, carbon tetrachloride, 1,2-dichloroethane, ethylene chloride, chlorobenzene, dichlorobenzene and the like; ethers such as ethyl ether, methyl ethyl ether, isopropyl ether, butyl ether, dioxane, dimethoxyethane (DME), tetrahydrofuran (THF), diethyleneglycol dimethylether (DGM) and the like; ketones such as acetone, methylethyl (MEK), methyl-isopropyl ketone, methyl-isobutyl ketone (MIBK) and the like; nitriles such as acetonitrile, propionitrile, acrylonitrile, and the like; esters such as, for example, ethyl acetate, amyl acetate and the like; acid amides such as dimethyl formamide (DMF), dimethyl acetamide (DMA), N-methylpyrrolidone, 1,3-dimethyl-2-imidazolidinone, hexamethylphosphoric triamide (HMPA) and the like; sulfones and sulfoxides such as dimethyl sulfoxide (DMSO), sulfolane and the like, and bases such as pyridine for example.

The above-mentioned process e) is carried out in the presence of an acid binder and as the acid binder may be mentioned inorganic bases such as, for example, hydroxide, carbonate, and bicarbonate of alkalimetals such as, for example, sodium hydrogen carbonate, potassium hydrogen carbonate, sodium carbonate, potassium carbonate, lithium hydroxide, sodium hydroxide, potassium hydroxide, and calcium

hydroxide. As inorganic alkalimetal amides may be mentioned, for example, lithium amide, sodium amide, and potassium amide, for example. As organic bases may be mentioned tertiary amines, dialkylaminoanilines and pyridines such as, for example, triethylamine, 1,1,4,4-tetramethylethylenediamine (TMEDA), N,N-dimethylaniline, N,N-diethylaniline, pyridine, 4-dimethylamino pyrimidine (DMAP), 1,4-diazabicyclo[2,2,2]-octane (DABCO), and 1,8-diazabicyclo[5,4,0]undec-7-ene (DBU).

Further may be mentioned organic lithium compounds such as, for example, methyl lithium, n-butyl lithium, sec-butyl lithium, tert-butyl lithium, phenyl lithium, dimethylcopper lithium, lithium diisopropylamide, lithium cyclohexylisopropylamide, lithium dicyclohexylamide, n-butyl lithium, DABCO, n-butyl lithium, DBU, and n-butyl lithium, TMEDA, etc.

In the above-mentioned process e), the reaction temperature can be varied within a substantially wide range. In general, the reaction is carried out at a temperature of from about -10 °C to about 100 °C, preferably from about 0 °C to about 35 °C.

Further, the reaction is carried out under normal pressure, although it is also possible to employ a higher or reduced pressure.

When the above-mentioned process e) according to the present invention is carried out, use is made, for example, of about 1 to 2 mols amount of a compound represented by the general formula (V), in a diluent such as pyridine per 1 mol of a compound represented by the general formula (Ia) in the presence of an acid binder such as triethyl amine, for example, to obtain the aimed compound.

In carrying out the process f) mentioned above, use may be made, as suitable diluent, of any inert organic solvent.

Examples of such diluents are aliphatic, cycloaliphatic and aromatic, optionally chlorinated, hydrocarbons such as pentane, hexane, cyclohexane, petroleum ether, ligroin, benzene, toluene, xylene, dichloromethane, chloroform, carbon tetrachloride, 1,2,dichloroethane, ethylene chloride, chlorobenzene, dichlorobenzene and the like; ethers such as diethyl ether, methyl ethyl ether, isopropyl ether, butyl ether, dioxane, dimethoxyethane (DME), tetrahydrofuran (THF), diethyleneglycol dimethylether (DGM) and the like; ketone such as acetone, methylethyl ketone (MEK), methyl-isopropyl ketone, methylisobutyl ketone (MIBK) and the like; nitriles such as acetonitrile, propionitrile, acrylonitrile, and the like; esters such as, for example, ethyl acetate, amyl acetate and the like; acid amides such as dimethyl formamide (DMF), dimethyl acetamide (DMA), N-methylpyrrolidone, 1,3-dimethyl-2-imidazolidinone, hexamethylphosphoric triamide (HMPA) and the like; sulfones and sulfoxides such as dimethyl sulfoxide (DMSO), sulfolane and the like, and bases such as pyridine for example.

The above-mentioned process f) is carried out in the presence of an acid binder and as the acid binder may be mentioned inorganic bases such as, for example, hydroxide, carbonate and bicarbonate of alkalimetals such as, for example, sodium hydrogen carbonate, potassium hydrogen carbonate, sodium carbonate, potassium carbonate, lithium hydroxide, sodium hydroxide, potassium hydroxide, and calcium hydroxide. As inorganic alkalimetal amides may be mentioned, for example, lithium amide, sodium amide, and potassium amide, for example. As organic bases may be mentioned tertiary amines, dialkylaminoanilines and pyridines such as, for example, triethylamine, 1,1,4,4-tetramethylethylenediamine (TMEDA), N,N-dimethylaniline, N,N-diethylaniline, pyridine, 4-dimethylamino pyrimidine (DMAP), 1,4-diazabicyclo[2,2,2]-octane (DABCO), and 1,8-diazabicyclo[5,4,0]undec-7-ene (DBU).

Further may be mentioned organic lithium compounds such as, for example, methyl lithium, n-butyl lithium, sec-butyl lithium, tert-butyl lithium, phenyl lithium, dimethylcopper lithium, lithium diisopropylamide, lithium cyclohexylisopropylamide, lithium dicyclohexylamide, n-butyl lithium, DABCO, n-butyl lithium, DBU, and n-butyl lithium, TMEDA, etc.

In the above-mentioned process f), the reaction temperature can be varied within a substantially wide range. In general, the reaction is carried out at a temperature of from about -10 °C to about 100 °C, preferably from about 0 °C to about 35 °C.

Further, the reaction is carried out under normal pressure, although it is also possible to employ a higher or reduced pressure.

When the above-mentioned process f) according to the present invention is carried out, use is made, for example, of about 1 to 2 mols amount of a compound represented by the general formula (VI), in a diluent such as pyridine per 1 mol of a compound represented by the general formula (Ia) in the presence of an acid binder, such as triethyl amine, for example, to obtain the aimed compound.

In carrying out the process g) mentioned above, use may be made, as suitable dilutent, of any inert organic solvent.

Examples of such diluents are aliphatic, cycloaliphatic and aromatic, optionally chlorinated, hydrocarbons such as pentane, hexane, cyclohexane, petroleum ether, ligroin, benzene, toluene, xylene, dichloromethane, chloroform, carbon tetrachloride, 1,2-dichloroethane, ethylene chloride, chlorobenzene, dichlorobenzene and the like; ethers such as diethyl ether, methyl ethyl ether, isopropyl ether, butyl ether, dioxane, dimethoxyethane (DME), tetrahydrofuran (THF), diethylenglycol dimethylether (DGM) and the like; ketones such as acetone, methylethyl ketone (MEK), methyl-isopropyl ketone, methyl-isopropyl ketone (MIBK) and the like; nitriles such as acetonitrile, propionitrile, acrylonitrile, and the like; esters such as, for example, ethyl acetate, amyl acetate and the like; acid amides such as dimethyl formamide (DMF), dimethyl acetamide (DMA), N-methylpyrrolidone, 1,3-dimethyl-2-imidazolidinone, hexamethylphosphoric triamide (HMPA) and the like; sulfones and sulfoxides such as dimethyl sulfoxide (DMSO), sulfolane and the like, and bases such as pyridine for example.

The above-mentioned process g) is carried out in the presence of an acid binder and as the acid binder may be mentioned organic bases such as tertiary amines, dialkylaminoanilines and pyridines such as, for example, triethylamine, 1,1,4,4-tetramethyl-ethylenediamine (TMEDA), N,N-dimethylaniline, N,N-diethylaniline, pyridine, 4-dimethylamino pyrimidine (DMAP), 1,4-diazabicyclo[2,2,2]octane (DABCO), and 1,8-diazabicyclo[5,4,0]undec-7-ene (DBU).

In the above-mentioned process g), the reaction temperature can be varied within a substantially wide range. In general, the reaction is carried out at a temperature of from about -30 °C to about 100 °C, preferably from about 0 °C to about 35 °C.

Further, the reaction is carried out under normal pressure, although it is also possible to employ a higher or reduced pressure.

When the above-mentioned process g) according to the present invention is carried out, use is made, for example, of about 1 to 4 mols of a compound (VII), in a diluent such as pyridine per 1 mol of a compound represented by the general formula (Ia) in the reaction to obtain the aimed compound.

The compounds according to the present invention act as either selective or non-selective herbicides depending on their concentration to be employed.

The active compounds according to the present invention may be used, for example, in the case of the following plants:

Dicotyledon weeds such as mustard (Sinapis), cress (Lepidium), bed straw (Galium), chickweed (Stellaria), goosefoot (Chenopodium album), annual nettle (Urtica), groundsell (Senecio), pigweed (Amaranthus), purslane (Portulaca), cocklebur (Xanthium), morning glory (Ipomoea), knotweed (Polygonum), ragweed (Ambrosia), spear thistle (Cirsium), sow thistle (Sonchus), egg plant, potato (Solanum), field cress (Rorippa), deadnettle (Lamium), speedwell (Veronica), thornapple (Datura), violet (Viola), hemp-nettle (Galeopsis), poppy (Papaver), knapweed (Centaurea), gallant soldier (Galinsoga), toothcup (Rotala) and false pimpernel (Lindernia) and like;

dicotyledon cultures such as cotton (Gossypium) soy bean (Glycine), beet (Beta), carrot (Daucus), bean (Phaseolus), pea (Pisum), potato (Solanum), flax (Linum), sweet potato (Ipomoe), broad bean (Vicia), tobacco (Nicotiana), tomato (Licopersicon), groundnut (Arachis), cabbage (Brassica), lettuce (Lactuca), cucumber (Cucumis) and marrow (Cucurbita);

monocotyledon weeds such as barnyard grass (Echinochloa), foxtail (Setaria), wild millet (Panicum), crabgrass (Digitaria), timothy (Phleum), bluegrass (Poa), fescue (Festuca), goosegrass (Eleusine), ryegrass (Lolium), cheat (Bromus), oats (Avena), flatsedge (Cyperus), sorghum (Sorghum), quackgrass (Agropyron), monochoria (Monochoria), fimbristylis (Fimbristylis), arrowhead (Sagittaria), spikerush (Eleocharis), bulrush (Scirpus), paspalum (Paspalum), Ischaemum, redtop (Agrostis), meadow foxtail (Alopecurus), and Bermuda grass (Cynodon); and

monocotyledon cultures such as rice (Oryza), maize (Zea), wheat (Triticum), barley (Hordeum), oats (Avena), rye (Secale), sorghum (Sorghum), millet (Panicum), sugar cane (Saccharum), pineapple (Ananas), asparagus (Asparagus) and onion (Allium).

However, the use of the active compounds according to the present invention is in no way restricted to these plants indicated above but embraces other plants, in the same way.

Depending on the concentration, the active compounds can be used for non-selective control of weeds, for example, on industrial terrain and railway tracks and on paths and squares with and without trees. Equally, the compounds can be employed for combating weeds in perennial cultures, for example, forestry plantings, decorative tree plantings, orchards, vineyards, citrus groves, nut orchards, banana plantations, coffee plantations, tea plantations, rubber plantations, oil palm plantations, cacao plantations, soft fruit plantings and hopfields, and for the selective controlling of weeds in annual cultures.

The active compounds according to the present invention can be converted into the customary formulations, such as liquid formulations, solutions, emulsions, suspensions, powders, wettable powders, dusting agents, foams, pastes, granules, aerosols, natural and synthetic materials impregnated with active compounds, microcapsules, coating compositions for use on seeds, and formulations used with burning equipment, such as fumigating cartridges, fumigating cans and fumigating coils, as well as ULV cold mist

and warm mist formulations.

These formulations may be produced in the manner known in themselves, for example, by mixing the active compounds with extenders, that is to say liquid or liquefied gaseous or solid diluents or carriers, optionally with the use of surface-active agents, that is to say emulsifying agents and/or dispersing agents and/or foam-forming agents. In the case of the use of water as an extender, organic solvents can, for example, also be used as auxiliary solvents.

As liquid diluents or carriers may be mentioned, in general, aromatic hydrocarbons, such as xylene, toluene or alkyl naphthalenes, chlorinated aromatic or chlorinated aliphatic hydrocarbons, such as chlorobenzenes, chloroethylenes or methylene chloride, aliphatic or alicyclic hydrocarbons, such as cyclohexane or paraffins, for example, mineral oil fractions, alcohols, such as butanol or glycol as well as their ethers and esters, ketones, such as acetone, methylethyl ketone, methylisobutyl ketone or cyclohexanone, for example, or strongly polar solvents, such as dimethylformamide and dimethylsulfoxide, as well as water.

By liquefied gaseous diluents or carriers are meant liquids which would be gaseous at normal temperature and under normal pressure, such as, for example, butane, propane, nitrogen and carbon dioxide, and aerosol propellants, such as halogenated hydrocarbons.

As solid carriers there may be used ground natural minerals, such as kaoline, clay, talc, chalk, quartz, attapulgite, montmorillonite or diatomaceous earth, etc., and ground synthetic minerals, such as highly-dispersed silicic acid, alumina and silicates, and the like.

As solid carriers for granules may be mentioned crushed and fractionated natural rocks such as calcite, marble, pumice, sepiolite and dolomite, as well as synthetic granules or inorganic and organic meals, and granules of organic material such as sawdust, coconut shells, maize cobs and tobacco stalks.

As emulsifying and/or foam-forming agents may be mentioned non-ionic and anionic emulsifiers, such as polyoxyethylene-fatty acid esters, polyoxyethylene-fatty alcohol ethers, for example, alkylaryl polyglycol ethers, alkyl sulfonates, alkyl sulfates, aryl sulfonates as well as albumin hydrolysis products.

Dispersing agents include, for example, lignin sulfite waste liquors and methylcellulose.

Adhesives such as carboxymethylcellulose and natural and synthetic polymers in the form of powders, granules or emulsions, such as, for example, gum arabic, polyvinyl alcohol and polyvinyl acetate, can be used in the formulations.

It is possible to use colorants such as inorganic pigments, for example, iron oxide, titanium oxide and Prussian Blue, and organic dyestuffs, such as alizarin dyestuffs, azo dyestuffs or metal phthalocyanine dyestuffs, and to use a trace amount of nutrients, such as salts of iron, manganese, boron, copper, cobalt, molybdenum and zinc.

The formulations in general contain from 0.1 to 95 percent by weight of active compound, preferably from 0.5 to 90 percent by weight.

The active compound according to the present invention can be used as such or in the form of herbicidal compositions for controlling weeds. It is also possible to combine the present active compounds with other known herbicidal agents. The resulting mixture can be handled either in the form of ready-to-use formulations or in the form of other formulations which are generally called as tank-mix.

As the known herbicidal agents that can be combined with the present active compounds may be mentioned:

For weed controlling in rice plant cultivation:

4-amino-6-(1,1-dimethylethyl)-3-ethylthio-1,2,4-triazine-5(4H)-one,

1-amino-6-ethylthio-3-(2,2-dimethypropyl)-1,3,5-triazine-2,4(1H,3H)-dione and

N-(2-benzothiazolyl)-N,N'-dimethylurea, etc.

For weed controlling in sugar cane cultivation:

4-amino-3-methyl-6-phenyl-1,2,4-triazine-5(4H)-one, etc.

For weed controlling in soybean cultivation:

4-amino-6-(1,1-dimethylethyl)-3-methylthio-1,2,4-triazine-5(4H)-one, etc.

Surprisingly, several mixture formulations of the present active compounds also exhibit synergistic effect.

The present active compounds can be used as such, as their formulations or as the use forms prepared therefrom by further dilution thereof, such as in the forms of ready-to-use solutions, emulsions, suspensions, powders, wettable powders, pastes and granules.

They may be used in the customary manner, for example, by watering, spraying, atomizing, dusting, scattering, etc.

The present active compounds can be used either in the pre-, or post-emergence period of the plants. It is also possible to apply the active compounds into soil before the seeds of plants are sown.

The amount of concentration of active compound used can vary within a substantial range. It depends essentially on the nature of the desired effect. In general, the amounts used as herbicide are from about 0.001 to about 10 kg of active compound per hectare, preferably from about 0.01 to about 5 kg/ha.

The preparation and the use of the active compounds according to the present invention are illustrated by the following examples.

It should be noted that the scope of the invention is not limited anyway to the technical contents of the Examples.

[Examples]

Synthesis Example 1

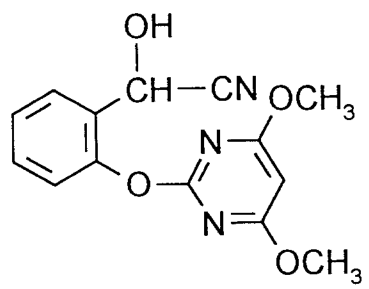

To a solution of 2-(4,6-dimethoxy-2-pyrimidinyloxy) benzaldehyde (16 g) in ether (100 ml) was dropwise added sodium hydrogen sulfite (21 g) dissolved in water (100 ml) at room temperature, followed by a one-hour stirring, and then further dropwise added potassium cyanate (13.6 g) dissolved in water (100 ml) followed by a violent stirring for a period of from 2 to 3 hours. The resulting organic layer was extracted several times with ether, followed by drying with anhydrous sodium sulfate, and removal of the solvent under reduced pressure distillation. The thus obtained residue was purified through a column chromatography (hexane/ethyl acetate) to obtain α-hydroxy-2-(4,6-dimethoxy-2-pyrimidinyloxy) phenylacetonitrile (18.1 g) having a m.p. in the range of from 109 to 111 °C.

Synthesis Example 2

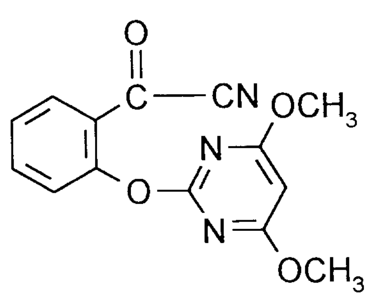

α-hydroxy-2-(4,6-dimethoxy-2-pyrimidinyloxy) phenylacetonitrile (1.7 g) was dissolved in acetone (30 ml), followed by a slow dropwise addition of 8N Jones reagent (0.8 ml) at 0 to 5 °C and then stirring of the resulting solution for a period of 12 hours. To the thus treated solution was added water (50 ml), followed by removal of the acetone under a reduced pressure distillation, then a few times of extraction with ethyl acetate, drying with anhydrous sodium sulfate, removal of the solvent, and purification of the residue through column chromatography (hexane/ethyl acetate), to obtain 2-(4,6-dimethoxy-2-pyrimidinyloxy) benzoylcyanide (1.0 g) having a melting point in the range of from 133 to 133.5 °C.

Synthesis Example 3

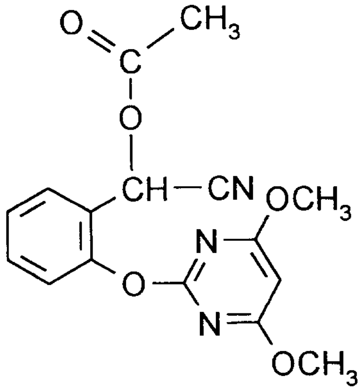

α-hydroxy-2-(4,6-dimethoxy-2-pyrimidinyloxy) phenylacetonitrile (1.6 g) was dissolved in pyridine (20 ml), followed by a dropwise addition of acetic anhydride (10 ml) thereto at room temperature. To the resulting solution, after a five-hour stirring, water was added (100 ml), followed by extraction with ethyl acetate, and the thus obtained organic layer was washed with a 2% hydrochloric acid, water, a saturated aqueous solution of sodium hydrogen carbonate, and water, in that order. the resulting mixture, after drying with anhydrous sodium sulfate, was removed from the solvent under a reduced pressure distillation and the residue was purified by column chromatography (hexane/ethyl acetate) to obtain α-acetoxy-2-(4,6-dimethoxy-2-pyrimidinyloxy) phenylacetonitrile (1.0 g), exhibiting $n_D^{20}$ = 1.5088.

Synthesis Example 4

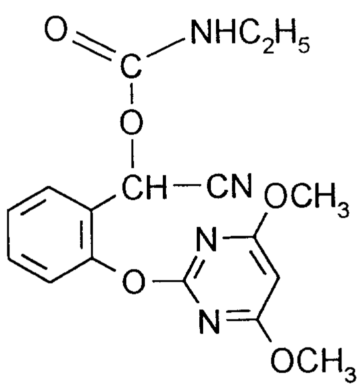

A mixture consisting of α-hydroxy-2-(4,6-dimethoxy-2-pyrimidinyloxy) phenylacetonitrile (1.6 g), ethyl isocyanate (0.5 g), a catalytic amount of triethylamine and tetrahydrofurane (50 ml) was stirred at room temperature for a period of from 5 to 6 hours. The resulting mixture was removed from the tetrahydrofurane under reduced pressure distillation, followed by addition of water (100 ml) thereto, an extraction with ethyl acetate, drying with anhydrous sodium sulfate, removal of the solvent under reduced pressure distillation and finally purification of the residue through column chromatography (hexane/ethyl acetate) to obtain α-ethylaminocarbonyloxy-2-(4,6-dimethoxy-2-pyrimidinyloxy)phenylacetonitrile (0.7 g), exhibiting $n_D^{20}$ = 1.5258.

Synthesis Example 5

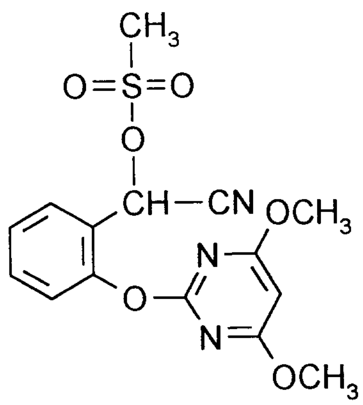

To a mixture consisting of α-hydroxy-2-(4,6-dimethoxy-2-pyrimidinyloxy) phenylacetonitrile (1.0 g), triethylamine (0.6 g) and methylene chloride (50 ml) was dropwise added methane sulfonyl chloride (0.6 g) at room temperature, followed by a further five-hour stirring. After the completion of reaction, the mixture was washed with water. The mixture, after drying with anhydrous sodium sulfate, was removed from the solvent under reduced pressure distillation, followed by purification of the resulting residue through column chromatography (hexane/ethyl acetate) to obtain α-methanesulfonyloxy-2-(4,6-dimethoxy-2-pyrimidinyloxy) phenylacetonitrile (0.6 g), exhibiting $n_D^{20}$ = 1.5281.

Synthesis Example 6

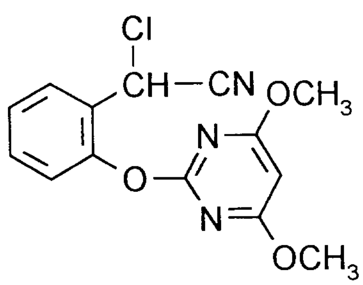

α-hydroxy-2-(4,6-dimethoxy-2-pyrimidinyl) phenylacetonitrile (1.0 g) was dissolved in pyridine (20 ml) and, to the resulting solution was slowly and dropwise added methanesulfonyl chloride (1.6 g) at 0 to 5°C. The thus treated solution was stirred for a period of one hour, while it was kept at the same temperature and then further stirred at room temperature for a period of 5 to 6 hours. After the completion of the reaction, water (100 ml) was added to the reaction mixture, followed by extraction with ethyl acetate and the thus obtained organic layer was washed with a 2% hydrochloric acid, water, a saturated aqueous solution of sodium hydrogen carbonate, and water, in that order. After drying with anhydrous sodium sulfate, the reaction product was removed from the solvent under reduced pressure distillation, followed by purification of the residue through column chromatography (hexane/ethyl acetate) to obtain α-chloro-2-(4,6-dimethoxy-2-pyrimidinyloxy) phenylacetonitrile (0.6 g), exhibiting $n_D^{20}$ = 1.5458.

Together with the compounds synthesized above, compounds as having been synthesized like the above-mentioned synthesis examples 1 to 6, are shown in the following Table 2:

Table 2

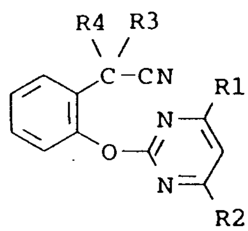

| Compoud No. | R1 | R2 | R3 | R4 | Physical constant |
|---|---|---|---|---|---|
| 1 | $OCH_3$ | $OCH_3$ | OH | H | mp. 109～111℃ |
| 2 | $OCH_3$ | $OCH_3$ | O | | mp. 133～133.5℃ |
| 3 | $OCH_3$ | $OCH_3$ | Cl | H | $n_D^{20}$ 1.5458 |
| 4 | $OCH_3$ | $OCH_3$ | 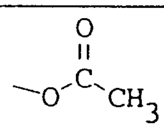 | H | $n_D^{20}$ 1.5088 |
| 5 | $OCH_3$ | $OCH_3$ | 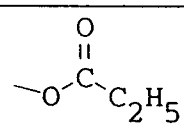 | H | $n_D^{20}$ 1.5258 |
| 6 | $OCH_3$ | $OCH_3$ | 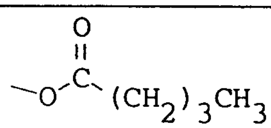 | H | $n_D^{20}$ 1.5127 |
| 7 | $OCH_3$ | $OCH_3$ | 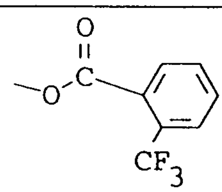 | H | mp. 83～86.5℃ |
| 8 | $OCH_3$ | $OCH_3$ | 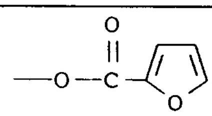 | H | $n_D^{20}$ 1.5406 |
| 9 | $OCH_3$ | $OCH_3$ | 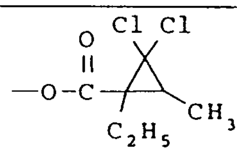 | H | $n_D^{20}$ 1.5273 |
| 10 | $OCH_3$ | $OCH_3$ | 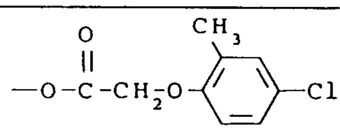 | H | $n_D^{20}$ 1.5638 |

Table 2

| Compound No. | R1 | R2 | R3 | R4 | Physical constant |
|---|---|---|---|---|---|
| 11 | $OCH_3$ | $OCH_3$ | 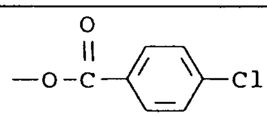 | H | mp. 109~112℃ |
| 12 | $OCH_3$ | $OCH_3$ | 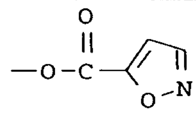 | H | $n_D^{20}$ 1.5259 |
| 13 | $OCH_3$ | $OCH_3$ | 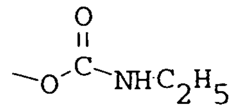 | H | $n_D^{20}$ 1.5258 |
| 14 | $OCH_3$ | $OCH_3$ | $-O-SO_2-CH_3$ | H | $n_D^{20}$ 1.5281 |
| 15 | $OCH_3$ | $OCH_3$ | 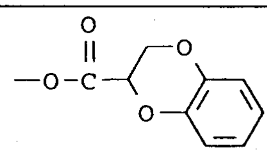 | H | $n_D^{20}$ 1.5378 |

Biotest Examples

Test Example 1

Pre-emergence soil treatment test on upland weeds

Formulation of Active Compounds

Carrier: 5 parts by weight of acetone
Emulsifier: 1 part by weight of benzyloxy polyglycol ether

In a greenhouse, a number of test pots each having an area of 120 $cm^2$ were charged with soil taken out from a cultivated field. Seeds of barnyard grass and wild amaranth were sown onto the soil surfaces in the respective test pots with each of the thus sown soil surfaces being covered with a soil layer.

Predetermined dosages of the active compound formulations as prepared like in the above-mentioned Test Example 1 were uniformly sprayed onto the soil surfaces of the test weeds in the respective test pots.

Four weeks after the spraying of the active compound formulations, the degrees of herbicidal effect on the weeds and phytotoxicity on crops were determined.

The resulting herbicidal effect was rated according to the following assessment scale:

| | |
|---|---|
| Completely killed | 100% |
| Status equivalent to non-treated pots | 0% |

In the present test, the compounds according to the present invention as identified above under Nos. 2, 3, 4, 5, 6, 10, 11 and 14, for example, each exhibited a 100% herbicidal effect at a dosage of 1.0 kg/ha on barnyard grass and wild amaranth.

Test Example 2

Post-emergence foliage treatment on upland weeds

Test Procedures

In a greenhouse, a number of test pots each having an area of 120 $cm^2$ were charged with soil taken out from a cultivated field. Seeds of barnyard grass and wild amaranth were sown onto the soil surfaces in the respective test pots with each of the thus sown soil surfaces being covered with a soil layer to allow them emerging.

After ten days (at the second leaf stage on average of the weeds), predetermined dosages of the active compound formulations prepared like the above-mentioned Test Example were uniformly sprayed onto the foliage portions of the test weeds in the respective test pots.

Three weeks after the spraying of the active compound formulations, the degrees of the herbicidal effect on the weeds and the degrees of the phytotoxicity on crops were determined.

In the present test, the compounds, according to the present invention as identified above under Nos. 4, 5, 6, 8 and 12, for example, exhibited a 100% herbicidal effect at a dosage of 1.0 kg/ha on barnyard grass and wild amaranth.

## Claims

**1.** Cyano-containing pyrimidine derivatives of the general formula (I)

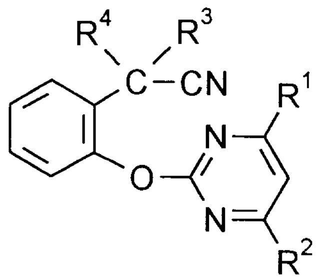

(I)

wherein

- $R^1$ represents halogen, $C_{1-4}$-alkyl, $C_{1-4}$-alkoxy, halogeno-$C_{1-4}$-alkyl, or halogeno-$C_{1-4}$-alkoxy,
- $R^2$ represents halogen, $C_{1-4}$-alkoxy, halogeno-$C_{1-4}$-alkyl, or halogeno-$C_{1-4}$-alkoxy,
- $R^3$ represents halogen, hydroxy, or an oxo group formed together with $R^4$ or
- $R^3$ represents one of the following groups represented by

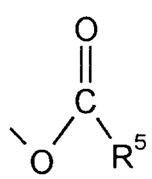 , 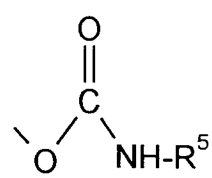

and -O-$SO_2$-$R^6$,

- $R^4$ represents hydrogen or an oxo group formed together with $R^3$,
- $R^5$ represents hydrogen or in each case an optionally substituted or unsubstituted $C_{1-20}$-alkyl, $C_{2-20}$-alkenyl, $C_{2-20}$-alkynyl, $C_{1-4}$-alkoxycarbonyl, $C_{3-7}$-cycloalkyl, phenyl, naphthyl or carboxyl or
- $R^5$ represents an optionally substituted or unsubstituted five- or six-membered heterocyclic ring, or an optionally substituted bicyclic group formed by condensation of a five or six-membered heterocyclic ring with phenyl, and
- $R^6$ represents in each case an optionally substituted or unsubstituted $C_{1-20}$-alkyl, $C_{2-20}$-alkenyl, $C_{2-20}$-alkynyl or phenyl.

**2.** Cyano-containing pyrimidine derivatives of the general formula (I) according to claim 1, characterized in that

$R^1$ represents halogen, $C_{1-3}$-alkyl, $C_{1-3}$-alkoxy, halogeno-$C_{1-3}$-alkyl or halogeno-$C_{1-3}$-alkoxy,
$R^2$ represents halogen, $C_{1-3}$-alkoxy, halogeno-$C_{1-3}$-alkyl or halogeno-$C_{1-3}$-alkoxy,
$R^3$ represents chloro, bromo, hydroxy, or an oxo group formed together with $R^4$ or
$R^3$ represents one of the following groups represented by

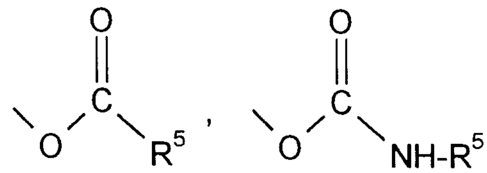

and $-O-SO_2-R^6$,
$R^4$ represents hydrogen, or an oxo group formed together with $R^3$,
$R^5$ represents hydrogen, $C_{1-15}$-alkyl, halogeno-$C_{1-4}$-alkyl, $C_{1-4}$-alkoxy-$C_{1-4}$-alkyl, $C_{3-7}$-cycloalkyl-$C_{1-4}$-alkyl, wherein said cycloalkyl may be substituted by $C_{1-4}$-alkyl or carboxyl, or
$R^5$ further represents cyano-$C_{1-4}$-alkyl, or phenyl-$C_{1-4}$-alkyl wherein said phenyl may be substituted by halogen or $C_{1-4}$-alkyl,
$R^5$ further represents phenoxy-$C_{1-4}$-alkyl, wherein said phenoxy may be substituted by halogen or a $C_{1-4}$-alkyl,
$R^5$ further represents phenylthio-$C_{1-4}$-alkyl wherein said phenylthio may be substituted by halogen, or $C_{1-4}$-alkyl,
$R^5$ further represents naphthyl-$C_{1-4}$-alkyl, carboxyl-$C_{1-4}$-alkyl, $C_{1-4}$-alkoxy-carbonyl-$C_{1-4}$-alkyl, or $C_{2-6}$-alkenyl wherein said alkenyl may be substituted by halogen or carboxyl,
$R^5$ further represents $C_{2-6}$-alkynyl, $C_{1-4}$-alkoxy-carbonyl wherein said alkoxy may be substituted by halogen, $C_{1-4}$-alkyl or carboxyl,
$R^5$ further represents $C_{3-7}$-cycloalkyl wherein said cycloalkyl may be substituted by halogen, $C_{1-4}$-alkyl or carboxyl,
$R^5$ further represents phenyl or naphthyl wherein said phenyl may be substituted by halogen, $C_{1-4}$-alkyl, halogeno-$C_{1-4}$-alkyl, $C_{1-4}$-alkoxy, halogeno-$C_{1-4}$-alkoxy, cyano, nitro or carboxyl,
$R^5$ further represents a five- or six-membered heterocyclic ring, wherein said hetero atom may be selected from the group consisting of oxygen, nitrogen and sulfur, while said heterocyclic ring may be substituted by halogen or $C_{1-4}$-alkyl,
$R^5$ further represents a group formed by condensation with a five- or six-membered heterocyclic ring with phenyl, wherein the hetero atom of said heterocyclic ring may be selected from the group consisting of oxygen, nitrogen and sulfur and said bicyclic group may be substituted by halogen, $C_{1-4}$-alkyl, $C_{1-4}$-alkylthio or carboxyl, and
$R^6$ represents a $C_{1-15}$-alkyl, halogeno-$C_{1-4}$-alkyl, $C_{1-4}$-alkoxy-$C_{1-4}$-alkyl, $C_{3-7}$-cycloalkyl-$C_{1-4}$-alkyl wherein said cycloalkyl may be substituted by $C_{1-4}$-alkyl or carboxyl,
$R^6$ further represents cyano-$C_{1-4}$-alkyl or phenyl-$C_{1-4}$-alkyl wherein said phenyl may be substituted by halogen or $C_{1-4}$-alkyl,
$R^6$ further represents phenoxy-$C_{1-4}$-alkyl wherein said phenoxy may be substituted by halogen or $C_{1-4}$-alkyl,
$R^6$ further represents phenylthio-$C_{1-4}$-alkyl, wherein said phenylthio may be substituted by halogen-$C_{1-4}$-alkyl,
$R^6$ further represents naphthyl-$C_{1-4}$-alkyl, carboxyl-$C_{1-4}$-alkyl, $C_{1-4}$-alkoxy-carbonyl-$C_{1-4}$-alkyl, $C_{2-6}$-alkenyl, wherein said alkenyl may be substituted by halogen or carboxyl, or
$R^6$ further represents $C_{2-6}$-alkynyl or phenyl.

**3.** Cyano-containing pyrimidino derivatives of the general formula (I) according to Claim 1, characterized in that

$R^1$ represents methoxy,
$R^2$ represents methoxy,
$R^3$ represents chloro, hydroxy, or an oxo group formed together with $R^4$ or
$R^3$ represents one of the following groups represented by

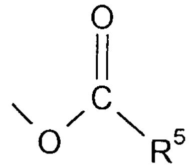 , 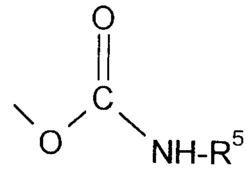

and $-O-SO_2-R^6$,

$R^4$ represents hydrogen or an oxo group formed together with $R^3$,

$R^5$ represents hydrogen, $C_{1-4}$-alkyl, fluoro-$C_{1-4}$-alkyl, chloro-$C_{1-4}$-alkyl, $C_{1-2}$-alkoxy-$C_{1-4}$-alkyl, $C_{3-6}$-cycloalkyl-$C_{1-4}$-alkyl, wherein said cycloalkyl may be substituted by methyl, ethyl, or carboxyl,

$R^5$ further represents cyano-$C_{1-4}$-alkyl, phenyl-$C_{1-4}$-alkyl wherein said phenyl may be substituted by chloro, bromo, or $C_{1-2}$-alkyl,

$R^5$ further represents phenoxy-$C_{1-4}$-alkyl wherein said phenoxy may be substituted by halogen or $C_{1-4}$-alkyl,

$R^5$ further represents phenylthio-$C_{1-4}$-alkyl wherein said phenylthio may be substituted by halogen or $C_{1-4}$-alkyl,

$R^5$ further represents naphthyl-$C_{1-4}$-alkyl, carboxyl-$C_{1-4}$-alkyl, $C_{1-4}$-alkoxy-carbonyl-$C_{1-4}$-alkyl, $C_{2-6}$-alkenyl wherein said alkenyl may be substituted by halogen or carboxyl,

$R^5$ further represents $C_{2-6}$-alkynyl, $C_{1-4}$-alkoxy-carbonyl, wherein said alkoxy may be substituted by halogen, $C_{1-4}$-alkyl or carboxyl,

$R^5$ further represents $C_{3-7}$-cycloalkyl wherein said cycloalkyl may be substituted by halogen, $C_{1-4}$-alkyl or carboxyl,

$R^5$ further represents phenyl or naphthyl wherein said phenyl may be substituted by halogen, $C_{1-4}$-alkyl, halogeno-$C_{1-4}$-alkyl, $C_{1-4}$-alkoxy, halogeno-$C_{1-4}$-alkoxy, cyano, nitro or carboxyl,

$R^5$ further represents a five- or six-membered heterocyclic ring wherein the hetero atom of said heterocyclic ring may be selected from the group consisting of oxygen, nitrogen and sulfur and said heterocyclic ring may be substituted by one of the following substituents: halogen, $C_{1-4}$-alkyl, $C_{1-4}$-alkylthio,

$R^5$ further represents a bicyclic group formed by condensation of a five- or six-membered heterocyclic ring with phenyl, wherein the hetero atom of said bicyclic group may be selected from the group consisting of oxygen, nitrogen and sulfur, and said bicyclic group may be substituted by halogen or $C_{1-4}$-alkyl,

$R^6$ further represents $C_{1-15}$-alkyl, halogeno-$C_{1-4}$-alkyl, $C_{1-4}$-alkoxy-$C_{1-4}$-alkyl, $C_{3-7}$-cycloalkyl-$C_{1-4}$-alkyl wherein said cycloalkyl may be substituted by $C_{1-4}$-alkyl, or carboxyl,

$R^6$ further represents cyano-$C_{1-4}$-alkyl, phenyl-$C_{1-4}$-alkyl, wherein said phenyl may be substituted by halogen or $C_{1-4}$-alkyl,

$R^6$ further represents phenoxy-$C_{1-4}$-alkyl wherein said phenoxy may be substituted by halogen or $C_{1-4}$-alkyl,

$R^6$ further represents phenylthio-$C_{1-4}$-alkyl, wherein said phenylthio may be substituted by halogen or $C_{1-4}$-alkyl,

$R^6$ further represents naphthyl-$C_{1-4}$-alkyl, carboxyl-$C_{1-4}$-alkyl, $C_{1-4}$-alkoxy-carbonyl-$C_{1-4}$-alkyl, or $C_{2-6}$-alkenyl, wherein said alkenyl may be substituted by halogen or carboxyl, or

$R^6$ further represents $C_{2-6}$-alkynyl or phenyl.

**4.** Process for the preparation of cyano-containing pyrimidine derivatives of the general formula (I)

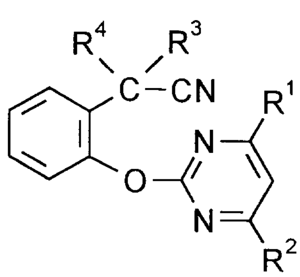

(I)

wherein, $R^1$, $R^2$, $R^3$, $R^4$ have the meanings as identified in claim 1, characterized in that they are obtained when

a) in the case where $R^3$ represents a hydroxyl group, and $R^4$ represents a hydrogen atom:
a compound represented by the general formula,

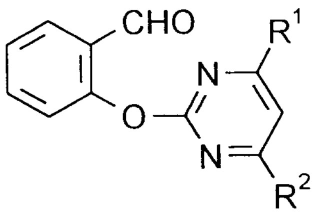

(II)

wherein
$R^1$ and $R^2$ have the same meanings as mentioned above,
is reacted with sodium cyanide or potassium cyanide, if appropriate in the presence of acidic catalysts and if appropriate in the presence of inert solvents,
or

b) in the case where $R^3$ and $R^4$ together form an oxo group: a compound represented by the general formula,

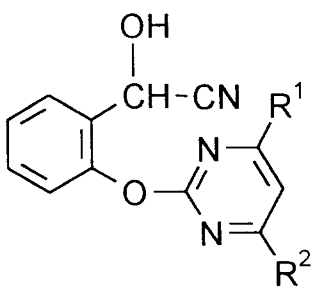

(Ia)

wherein
$R^1$ and $R^2$ have the same meanings as mentioned above,
is oxidized, if appropriate in the presence of inert solvents, or

c) in the case where $R^3$ represents $-O-SO_2-R^6$ and $R^4$ represents a hydrogen atom:
a compound represented by the formula (Ia) is reacted with a compound represented by the general formula

$R^6SO_2R^7$ (III)

wherein $R^6$ has the same meaning as mentioned above and $R^7$ represents an eliminable group such as chloro or bromo, if appropriate in the presence of acid binder and if appropriate in the presence of inert solvents,

or

d) in the case where $R^3$ represents

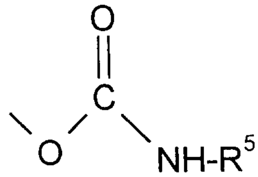

and $R^4$ represents a hydrogen atom:
a compound represented by formula (Ia) is reacted with a compound represented by the general formula

$R^5NCO$ (IV)

wherein $R^5$ has the same meaning as mentioned above, if appropriate in the presence of acid binder and if appropriate in the presence of inert solvents,

or

e) in the case where $R^3$ represents

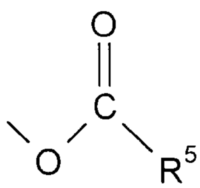

and $R^4$ represents a hydrogen atom: a compound represented by formula (Ia) is reacted with a compound represented by the general formula

$R^5COR^7$ (V)

wherein $R^5$ and $R^7$ have the same meanings as mentioned above, if appropriate in the presence of acid binder and if appropriate in the presence of inert solvents,

or

f) in the case where $R^3$ represents

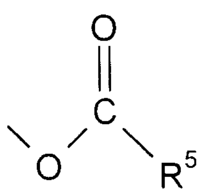

and $R^4$ represents a hydrogen atom: a compound represented by formula (Ia) is reacted with a compound represented by the general formula

$R^5COR^7$ (VI)

wherein $R^5$ and $R^7$ have the same meanings as mentioned above, if appropriate in the presence of acid binder and if appropriate in the presence of inert solvents,

or

g) in the case where $R^3$ represents chloro or bromo and $R^4$ represents a hydrogen atom: a compound represented by formula (Ia) is reacted with thionyl chloride, phosgene, oxal chloride, or a compound represented by the general formula

$R^5SO_2R^8$ (VII)

wherein $R^5$ has the same meanings as mentioned above, and $R^8$ represents chloro or bromo, if appropriate in the presence of acid binder and if appropriate in the presence of inert solvents.

**5.** Herbicidal compositions, characterized in that they contain at least one cyano-containing pyrimidine derivative of the general formula (I) according to claims 1 to 4.

**6.** Process for combating weeds, characterized in that cyano-containing pyrimidine derivatives of the general formula (I) according to claims 1 to 4 are allowed to act on weeds and/or their habitat.

7. Use of cyano-containing pyrimidine derivatives of the general formula (I) according to claims 1 to 4 for combating weeds.

8. Process for the preparation of herbicidal compositions, characterized in that cyano-containing pyrimidine derivatives of the general formula (I) according to claims 1 to 4, are mixed with extenders and/or surface active agents.